(19) Europäisches Patentamt
European Patent Office
Office européen des brevets

(11) EP 3 313 843 B1

(12) **EUROPEAN PATENT SPECIFICATION**

(45) Date of publication and mention of the grant of the patent:
**11.03.2020 Bulletin 2020/11**

(21) Application number: **16734826.7**

(22) Date of filing: **22.06.2016**

(51) Int Cl.:
***C07D 487/04*** *(2006.01)*

(86) International application number:
**PCT/US2016/038661**

(87) International publication number:
**WO 2016/209895 (29.12.2016 Gazette 2016/52)**

(54) **IMIDAZOPYRAZINE DERIVED COMPOUNDS FOR ELECTRONIC DEVICES**

IMIDAZOPYRAZIN-ABGELEITETE VERBINDUNGEN FÜR ELEKTRONISCHE VORRICHTUNGEN

COMPOSÉS DÉRIVÉS D'IMIDAZOPYRAZINE POUR DISPOSITIFS ÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2015 US 201562184948 P**

(43) Date of publication of application:
**02.05.2018 Bulletin 2018/18**

(73) Proprietors:
- **Dow Global Technologies LLC**
  **Midland, MI 48674 (US)**
- **Rohm And Haas Electronic Materials Korea Ltd.**
  **Chungcheongnam-do 331-980 (KR)**

(72) Inventors:
- **ONDARI, Mark E.**
  **Midland, MI 48674 (US)**
- **WELSH, Dean M.**
  **Midland, MI 48674 (US)**
- **FROESE, Robert DJ**
  **Midland, MI 48674 (US)**
- **NA, Hong-Yeop**
  **Cheonan 331-980 (KR)**

(74) Representative: **Houghton, Mark Phillip**
**Patent Outsourcing Limited**
**Cornerhouse**
**1 King Street**
**Bakewell**
**Derbyshire DE45 1DZ (GB)**

(56) References cited:
**WO-A1-2004/072081 WO-A1-2012/052745**
**CN-A- 104 650 088 CN-A- 104 650 089**
**CN-A- 104 650 116 CN-A- 104 672 240**
**US-A1- 2013 299 794 US-A1- 2014 364 414**

- **KAYAGIL, ISMAIL ET AL: "Synthesis of some 2,3,6,8-tetraarylimidazo[1,2-a]pyrazine derivatives by using either reflux or microwave irradiation method, and investigation their anticancer activities", TURKISH JOURNAL OF CHEMISTRY, vol. 35, no. 1, 2011, pages 13-24, XP002760886, ISSN: 1300-0527, DOI: 10.3906/KIM-1005-649**
- **GEMBUS, VINCENT ET AL: "Palladium Catalyzed One-Pot Sequential Suzuki Cross-Coupling-Direct C-H Functionalization of Imidazo[1,2-a]pyrazines", ORGANIC LETTERS, vol. 14, no. 23, 2012, pages 6012-6015, XP002760887, ISSN: 1523-7052, DOI: 10.1021/OL3029059**
- **GOEL, RICHA ET AL: "Palladium catalyzed novel monoarylation and symmetrical/unsymmetrical diarylation of imidazo[1,2-a]pyrazines and their in vitro anticancer activities", RSC ADVANCES, vol. 4, no. 19, 1 January 2014 (2014-01-01), pages 9885-9892, XP002760888, ISSN: 2046-2069, DOI: 10.1039/C3RA47192F**
- **SIDIR, ISA ET AL: "Conformational stability, the spectroscopic (FT-IR and UV), first order hyperpolarizability, NBO analysis, HOMO and LUMO analysis of 6,8-diphenylimidazo[1,2-.alpha.]pyrazine molecule by ab initio HF and density functional methods", SPECTROCHIMICA ACTA, PART A: MOLECULAR AND BIOMOLECULAR SPECTROSCOPY, vol. 81, no. 1, 2011, pages 339-352, XP002760889, ISSN: 1386-1425, DOI: 10.1016/J.SAA.2011.06.021**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- WANG, JIAN-XIN ET AL: "Palladium-Catalyzed Direct Heck Arylation of Dual .pi.-Deficient/.pi.-Excessive Heteroaromatics. Synthesis of C-5 Arylated Imidazo[1,5-a]pyrazines", ORGANIC LETTERS, vol. 10, no. 14, 1 January 2008 (2008-01-01), pages 2923-2926, XP002760890, ISSN: 1523-7060, DOI: 10.1021/OL800761R

## Description

REFERENCE TO RELATED APPLICATIONS

The present application claims the benefit of U.S. Provisional Application No. 62/184948, filed on June 26, 2015.

BACKGROUND

Organic electroluminescence (EL) devices are display devices that employ stacks of films containing organic aromatic compounds as electroluminescent layers. Such compounds are generally classified as electroluminescent materials and charge transport materials. Several properties, required for such electroluminescent and charge transport compounds, include high fluorescent quantum yield in solid state, high mobility of electrons and holes, chemical stability during vapor-deposition in vacuum, and the ability to form stable films. These desired features increase the lifetime of an EL device. There is a continual need for improved electroluminescent compounds and films containing the same.

The organic electroluminescent device generally has a structure comprising an anode, a cathode, and an organic layer between them. The organic layer comprises a light-emitting layer, and may further comprise at least one layer such as a hole injection layer, a hole transport layer, an electron injection layer, an electron transport layer, an interlayer, a hole blocking layer, and an electron blocking layer. Collectively, these components determine the overall device performance, such as external quantum efficiency and lifespan.

Desirable qualities for such self-lighting device architectures include low driving voltages, high charge transport capabilities of both holes and electrons, chemical stability during device fabrication and subsequent use, high luminescence efficiency, and long device lifetime. These addressable needs continue to be improved upon by device configurations that optimize both the device architecture as well as the individual components of the device stack.

U.S. Patent 7867629 discloses nitrogenous heterocyclic compounds for electroluminescent devices. U.S. Patent 7745016 (B2) discloses organic EL device emitting white light. U.S. Publication 20110227058 (A1) discloses an electroluminescent element, containing at least one layer containing at least one nitrogen-containing heterocyclic compound. Other compounds are disclosed in US7745016, EP406762A2 and JP2001043978A (Abstract). CN104650088 discloses novel carbazolyl-containing derivatives according to formulae 1-3 below.

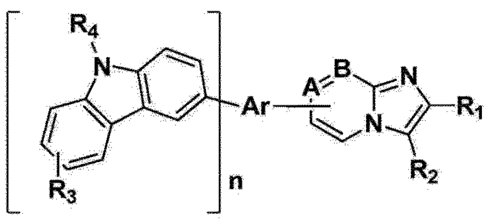

(1)

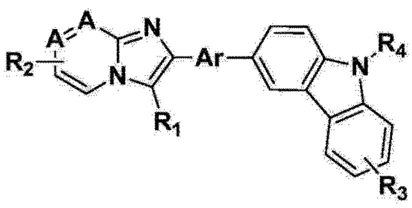

(2)

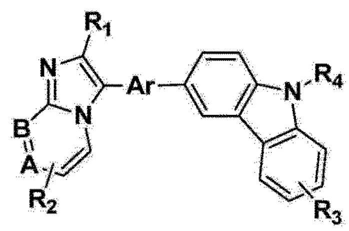

(3)

CN104650089 discloses novel fluorene derivatives containing a carbazole-3-yl group according to formulae 1-3 below.

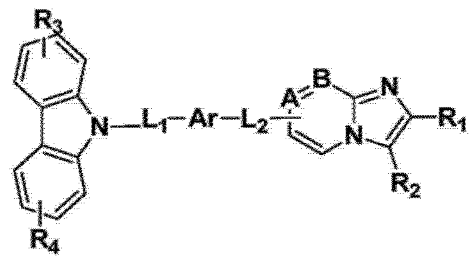

（1）

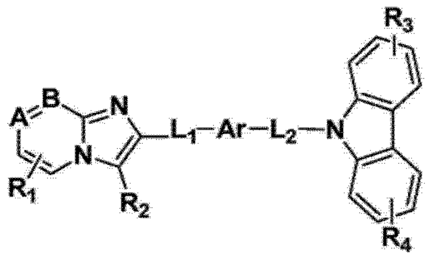

（2）

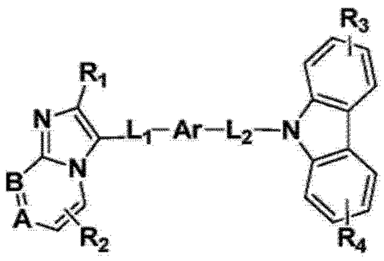

（3）

CN104672240 discloses novel carbazole type derivatives according to formulae 1-3 below.

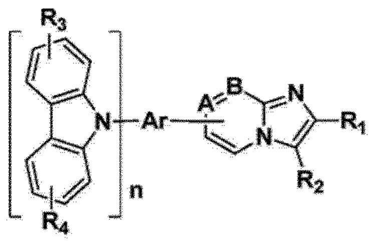

（1）

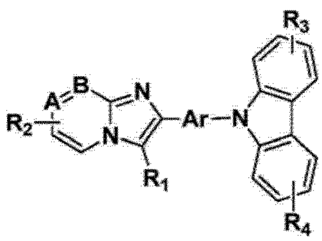

（2）

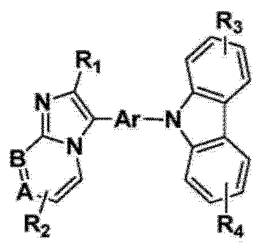

（3）

CN104650116 discloses organic compounds according to formula (I) below.

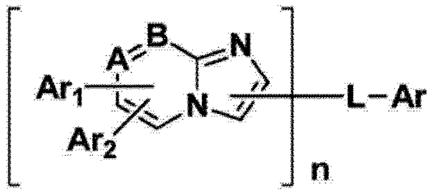

( I )

US2013/299794 discloses compounds for an organic optoelectronic device, according to Chemical Formula 1, below.

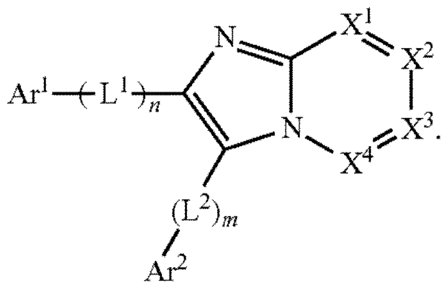

However, there is a continued need for new compounds which can used for organic electroluminescent devices having improved performance, and for an organic electroluminescent devices comprising the same. These needs have been met by the following invention.

SUMMARY OF INVENTION

The invention is set out in accordance with the appended claims. The invention provides a composition comprising at least one compound, in accordance with Formula 1 through Formula 8, selected from the following formulae (a) through (v1) as shown below:

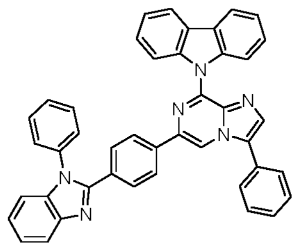

(a),

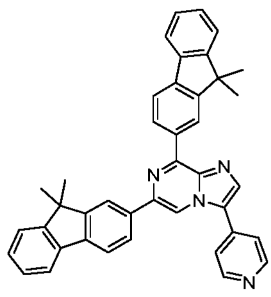

(b),

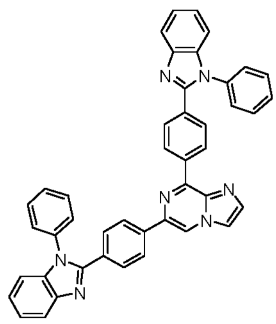

(c),

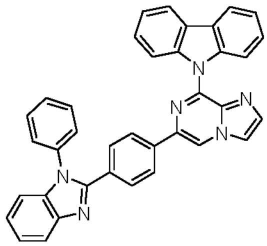 (d), 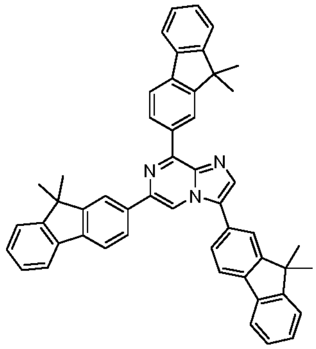 (e), 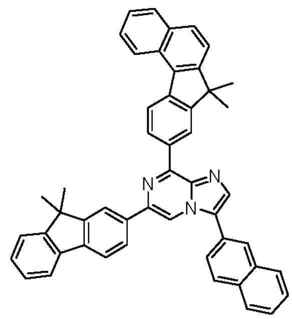 (f),

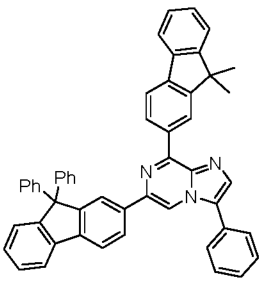 (g), 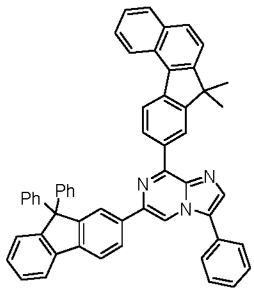 (h), 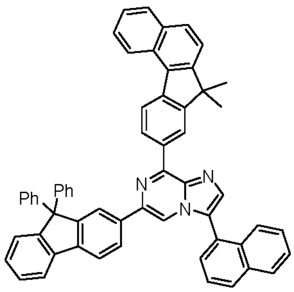 (i),

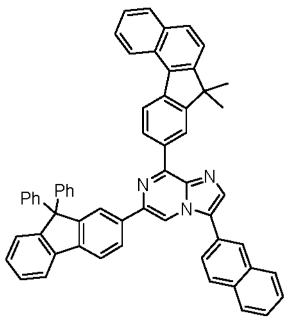 (j), 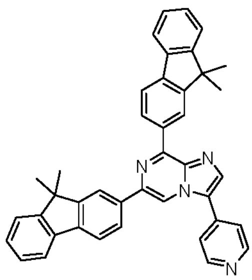 (k), 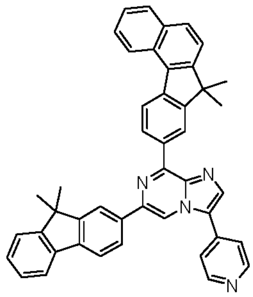 (l),

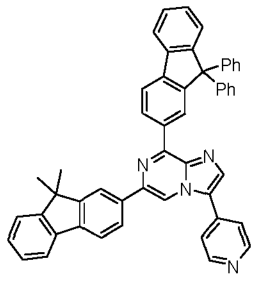 (m), 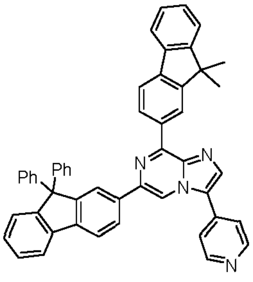 (n), 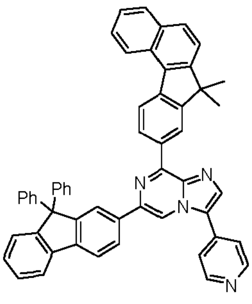 (o),

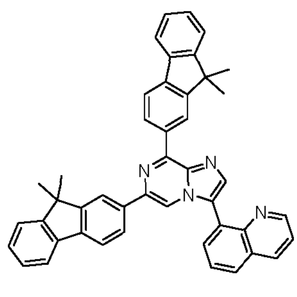 (p), 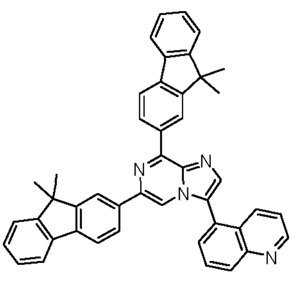 (q), 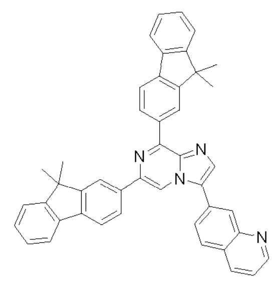 (r),

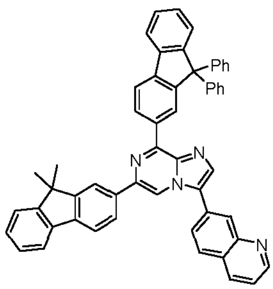 (s),

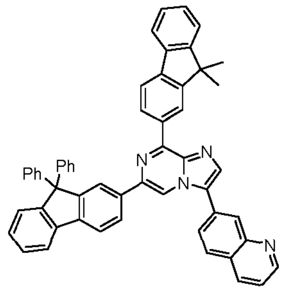 (t),

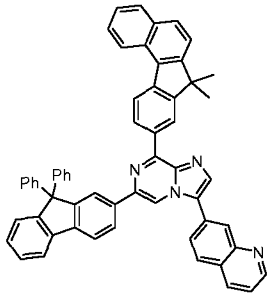 (u),

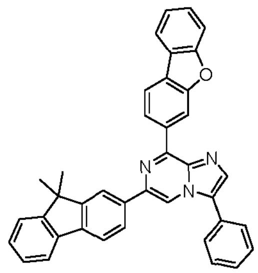 (v),

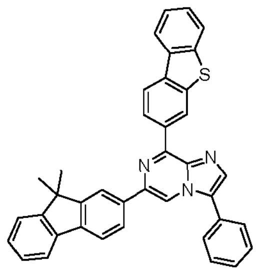 (w),

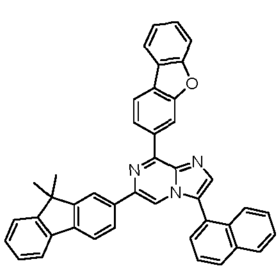 (x),

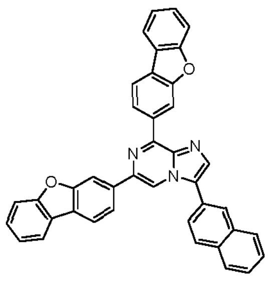 (y),

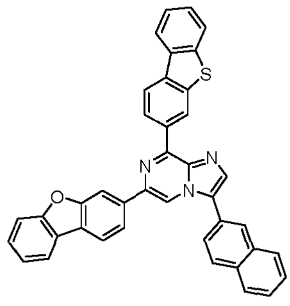 (z),

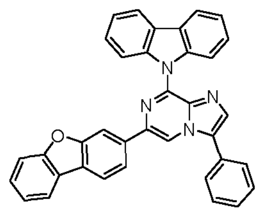 (a1),

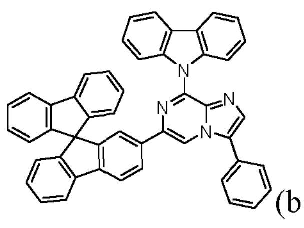 (b1),

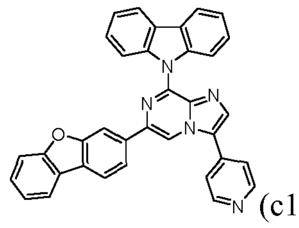 (c1),

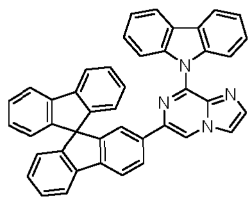 (d1),

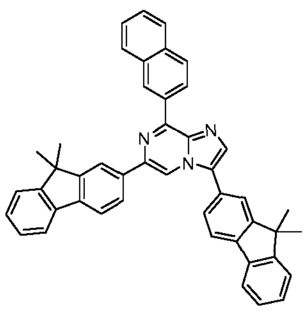 (e1),

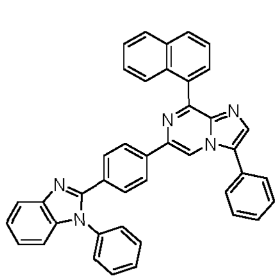 (f1),

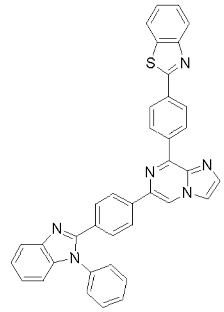 (g1),

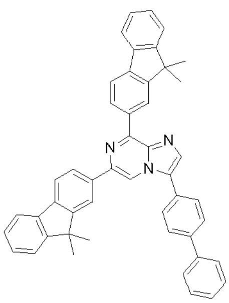 (h1), 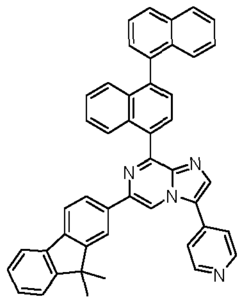 (i1), 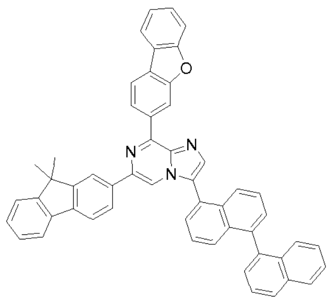 (j1),

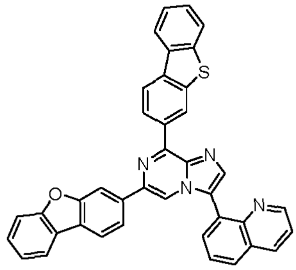 (k1), 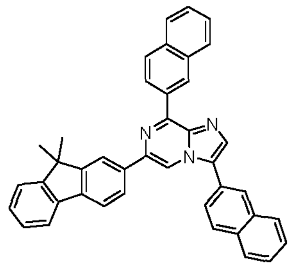 (l1), 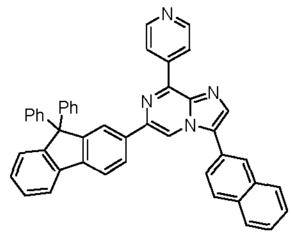 (m1),

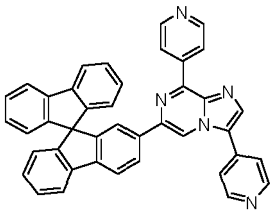 (n1), 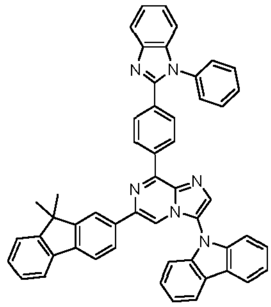 (o1), 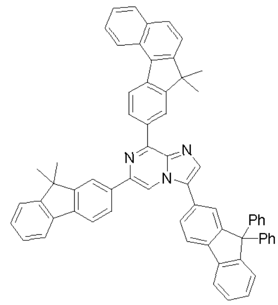 (p1),

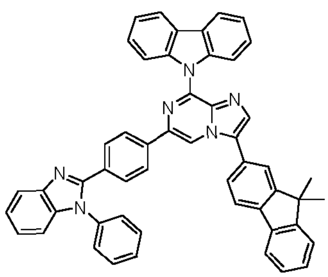 (q1), 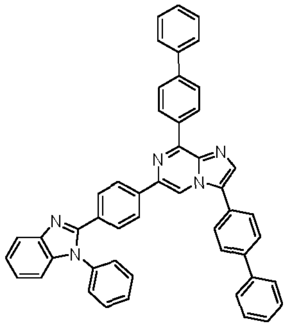 (r1), 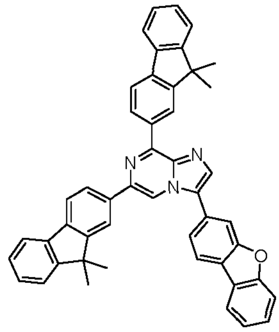 (s1),

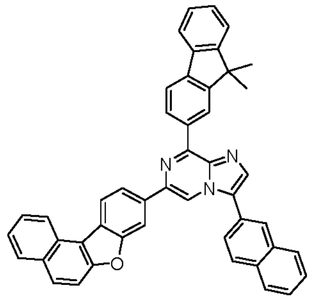 (t1), 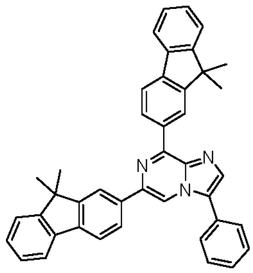 (u1), or 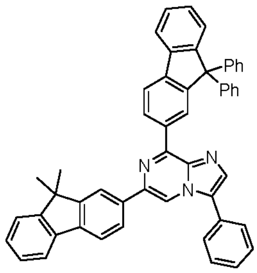

(v1).

DETAILED DESCRIPTION

Disclosed but not claimed are compounds of Formula 1 through Formula 8, as shown below:

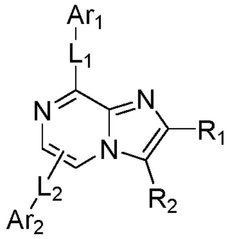 (Formula 1); 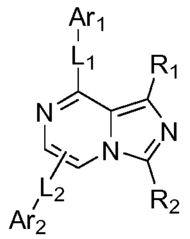 (Formula 2);

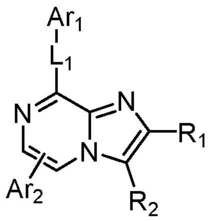 (Formula 3); 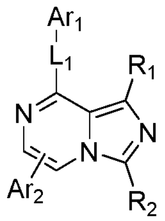 (Formula 4);

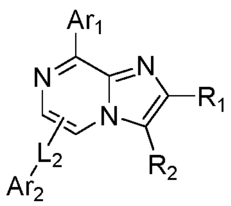 (Formula 5); 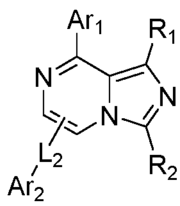 (Formula 6);

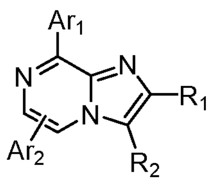 (Formula 7); 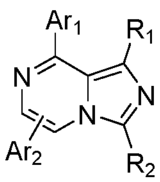 (Formula 8); and

wherein, for Formula 1, Formula 2, Formula 3, Formula 4, Formula 5, and Formula 6, independently, $R_1$ and $R_2$ are each, independently, selected from H, an alkyl, a substituted alkyl, a heteroalkyl, a substituted heteroalkyl, an aryl, a substituted aryl, a heteroaryl, or a substituted heteroaryl; and $L_1$ and/or $L_2$ are each, independently, a substituted or unsubstituted arylene or a substituted or unsubstituted heteroarylene; and $Ar_1$ and $Ar_2$ are each, independently, a substituted or unsubstituted aryl, or a substituted or unsubstituted heteroaryl; and
wherein, for Formula 7 and Formula 8, independently, $R_1$ and $R_2$ are each, independently, selected from H, an alkyl,

a substituted alkyl, a heteroalkyl, a substituted heteroalkyl, an aryl, a substituted aryl, a heteroaryl, or a substituted heteroaryl; and $Ar_1$ and $Ar_2$ are each, independently, a substituted or unsubstituted aryl, or a substituted or unsubstituted heteroaryl; and

wherein, for each of Formulas 1-Formula 8, $R_1$ and $R_2$ may, optionally, form one or more ring structures; and

wherein, for each of Formulas 1- Formula 8, one or more hydrogen atoms may, optionally, be replaced by deuterium.

As used herein, for each Formula 1-8, R1 = $R_1$, R2 = $R_2$, and so on.

In one embodiment, for each of the Formula 1, Formula 2, Formula 3, Formula 4, Formula 5, and Formula 6, independently, $L_1$ and/or $L_2$, are each, independently, an unsubstituted (3-to 30-membered)heteroarylene, a substituted (3- to 30-membered)hetero-arylene, an unsubstituted (C6-C30)arylene, or a substituted (C6-C30)arylene.

In one embodiment, for each of the Formula 1, Formula 2, Formula 3, Formula 4, Formula 5, and Formula 6, independently, $L_1$ and/or $L_2$, are each, independently, selected from one of the following structures:

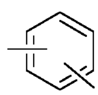 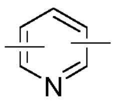 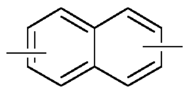 .

In one embodiment, for Formulas 1 through Formula 8, independently, $Ar_1$ and $Ar_2$, are each, independently, selected from an unsubstituted (3- to 30-membered) heteroarylene, a substituted (3- to 30-membered)heteroarylene, an unsubstituted (C6-C30)arylene, or a substituted (C6-C30)arylene.

In one embodiment, for Formulas 1 through Formula 8, independently, $Ar_1$ and $Ar_2$, are each, independently, selected from the following A1) to A48):

A1) 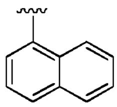, A2) 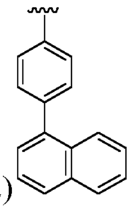, A3) 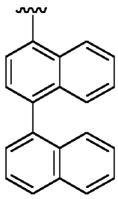, A4) 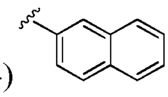, A5) 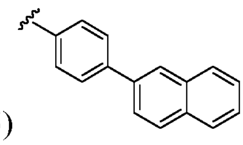,

A6) 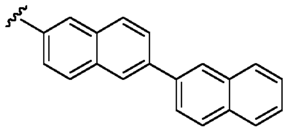, A7) 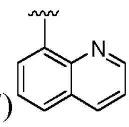, A8) 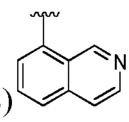, A9 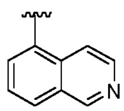, A10) 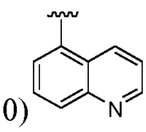,

A11) 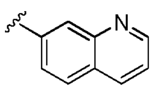, A12) 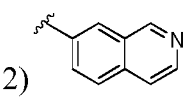, A13) 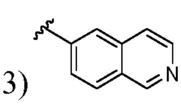, A14) 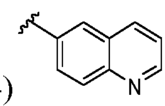,

A15) 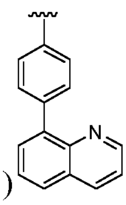, A16) 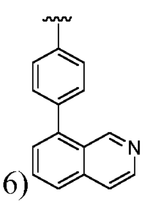, A17) 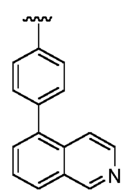, A18) 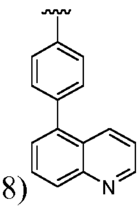, A19) 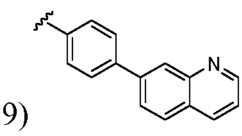,

A20) 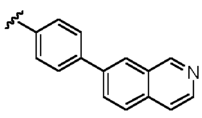, A21) 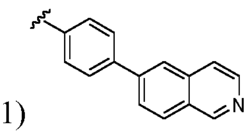, A22) 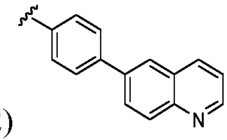, A23) 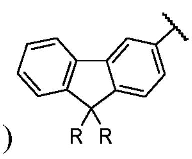,

A24) 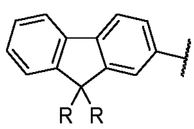, A25) 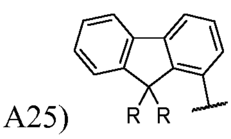, A26) 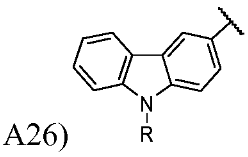, A27) 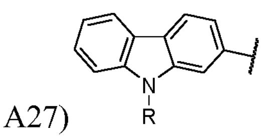,

A28) 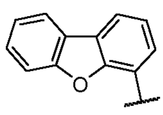 A29) 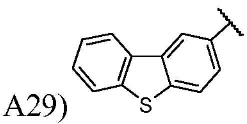, A30) 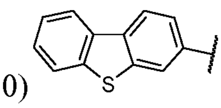, A31) 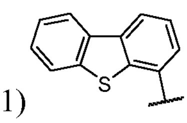,

A32) 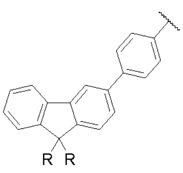, A33) 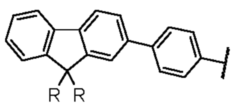, A34) 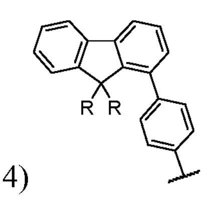, A35) 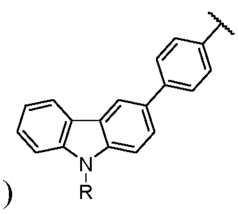,

A36) 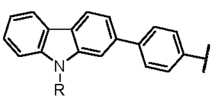, A37) 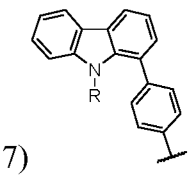, A38) 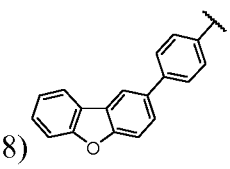, A39) 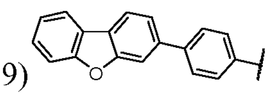,

A40) 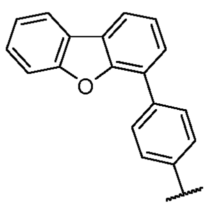, A41) 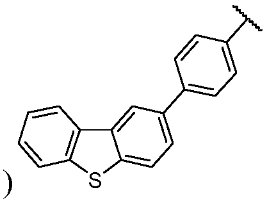, A42) 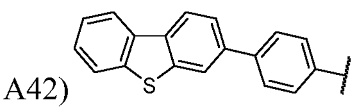,

A43) 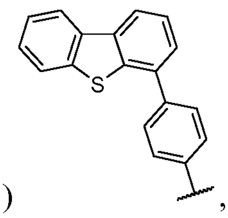, A44) 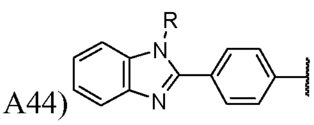, A45) 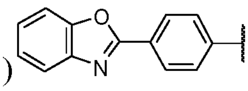, A46)

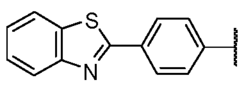, A47) 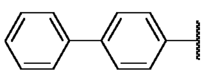, or A48) 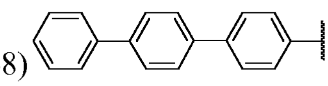.

For the above structures and other structures noted herein, the external connection point of each substituent is indicated by a wavy line, as recommended by current IUPAC standards: Pure Appl. Chem., 2008, 80, 277 (Graphical representation standards for chemical structural diagrams).

In one embodiment, for Formulas 1 through 8, independently, $R_1$ and $R_2$, are each, independently, selected from the following:

hydrogen,

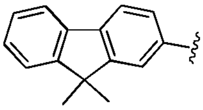, 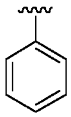, 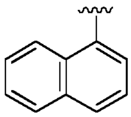, 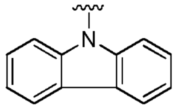, 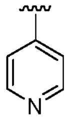 or 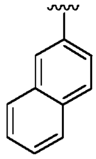.

Specifically, the invention provides a composition comprising at least one compound, in accordance with Formula 1 through Formula 8, selected from the following formulae (a) through (v1):

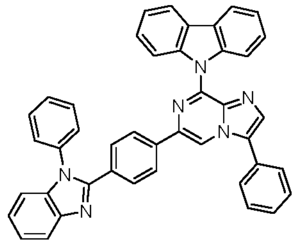 (a), 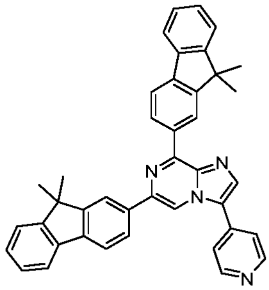 (b), 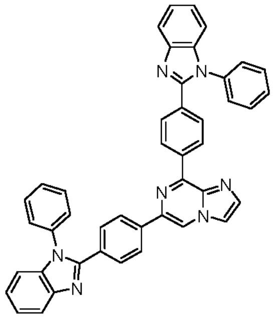 (c),

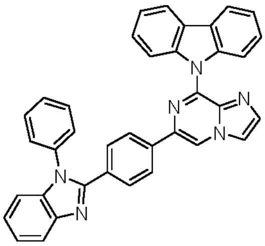 (d), 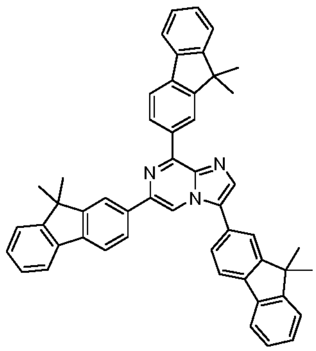 (e), 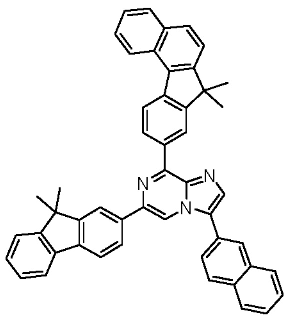 (f),

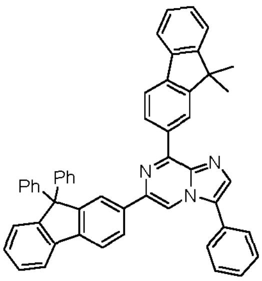 (g), 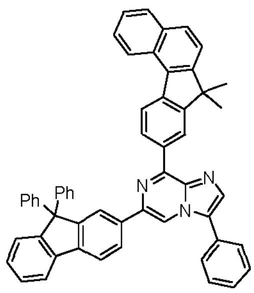 (h), 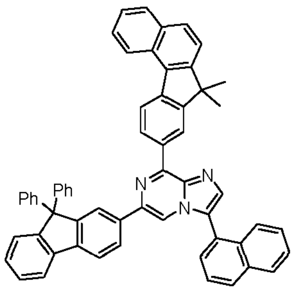 (i),

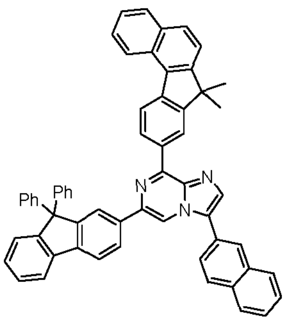 (j), 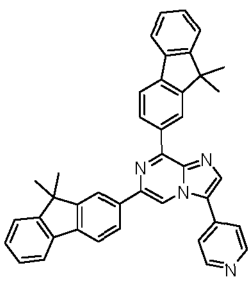 (k), 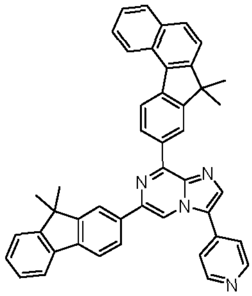 (l),

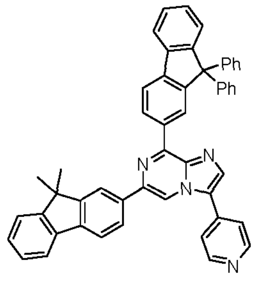 (m),

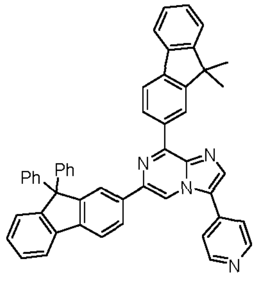 (n),

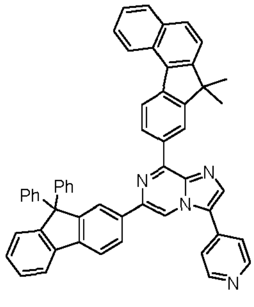 (o),

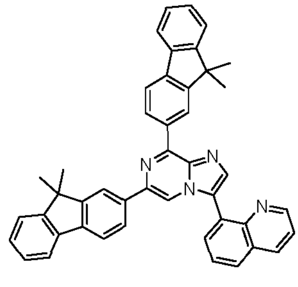 (p),

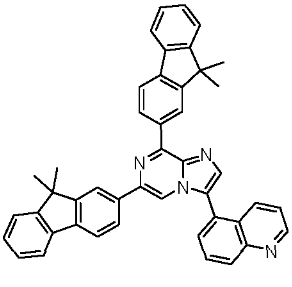 (q),

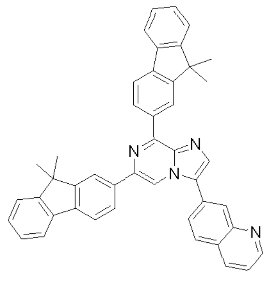 (r),

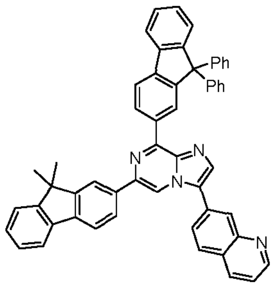 (s),

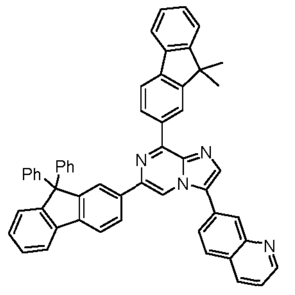 (t),

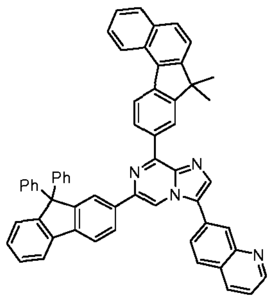 (u),

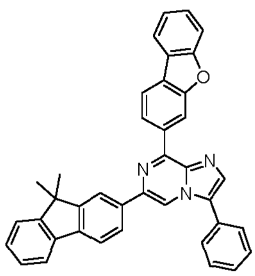 (v),

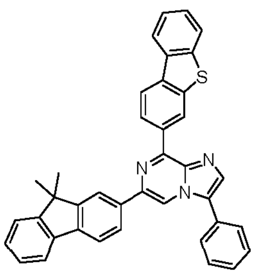 (w),

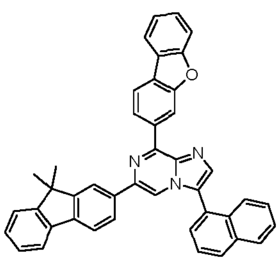 (x),

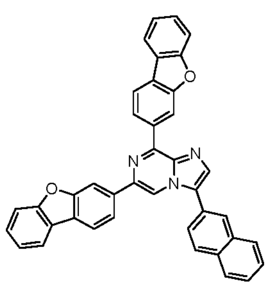 (y),

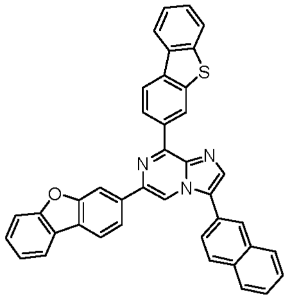 (z),

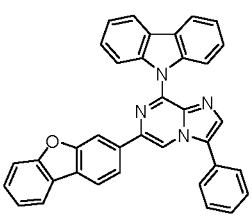 (a1),

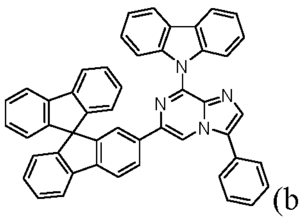 (b1), 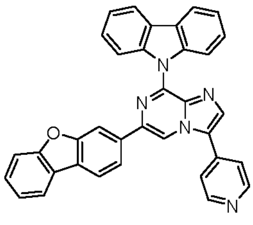 (c1), 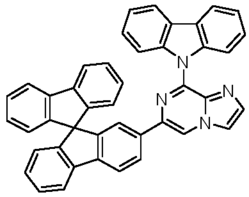 (d1),

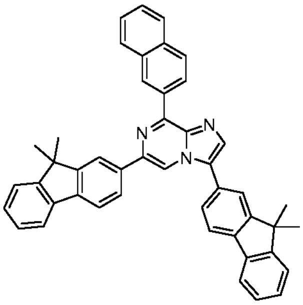 (e1), 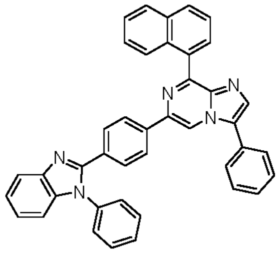 (f1), 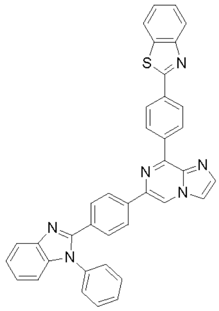 (g1),

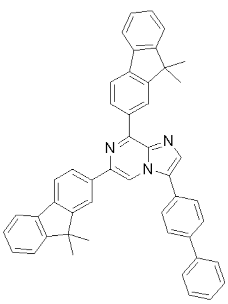 (h1), 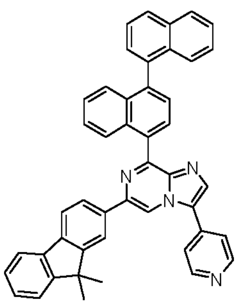 (i1), 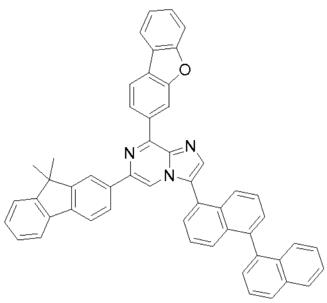 (j1),

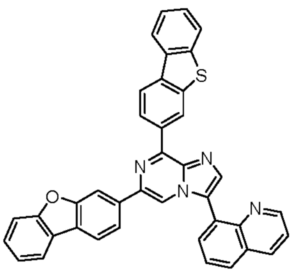 (k1), 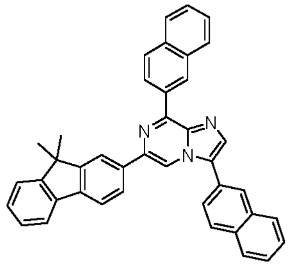 (l1), 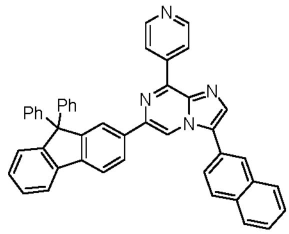 (m1),

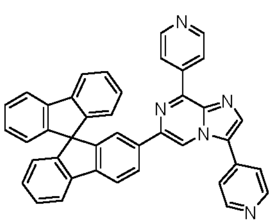 (n1), 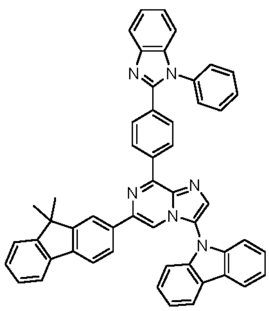 (o1), 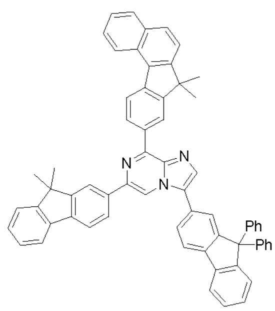 (p1),

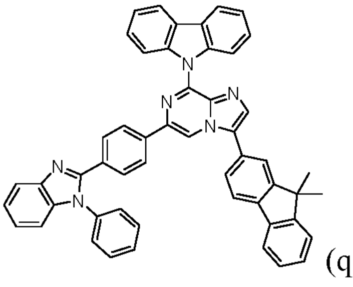 (q1), 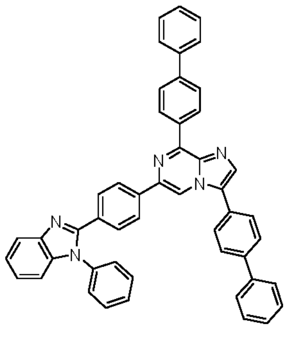 (r1), 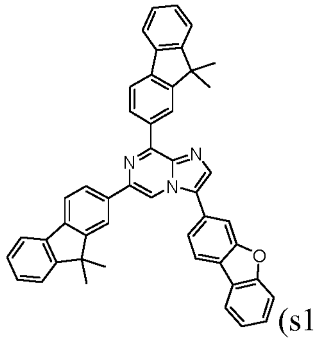 (s1),

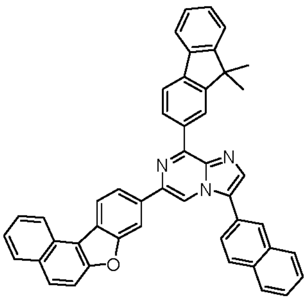 (t1), 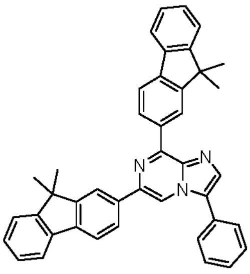 (u1), or 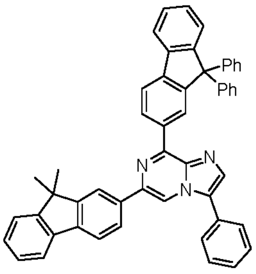 (v1),

The compound of the present invention can be prepared by synthetic methods known to one skilled in the art, such as oxidative cyclization, Suzuki coupling, among others.

In one embodiment, the compound has a molecular weight greater than, or equal to, 400 g/mole, or greater than, or equal to, 450 g/mole, or greater than, or equal to, 500 g/mole.

In one embodiment, the compound has a molecular weight from 400 to 900 g/mole, or from 450 to 850 g/mole.

In one embodiment, the compound has a HOMO level from -5.00 to -6.50 eV.

In one embodiment, the compound has a LUMO level from -1.60 to -2.10 eV.

In one embodiment, the compound has a glass transition temperature (Tg) from 105°C to 170°C.

In one embodiment, the composition further comprises a metal quinolate. In one embodiment, the metal quinolate contains at least one atom of deuterium. In a further embodiment, the metal quinolate is lithium quinolate.

In one embodiment, the composition comprises from 10 to 90 weight percent of the metal quinolate (for example, lithium quinolate), based on the weight of the composition. In a further embodiment, the composition comprises from 10 to 80, further from 10 to 70, further from 10 to 60, further from 10 to 50, weight percent of the metal quinolate (for example, lithium quinolate), based on the weight of the composition. In a further embodiment, the composition comprises from 20 to 50 weight percent of the metal quinolate (for example, lithium quinolate), based on the weight of the composition.

In one embodiment, the composition comprises from 10 to 90 weight percent of the metal quinolate (for example, lithium quinolate), based on the sum weight of an inventive compound and the metal quinolate. In a further embodiment, the composition comprises from 10 to 80, further from 10 to 70, further from 10 to 60, further from 10 to 50, weight percent of the metal quinolate (for example, lithium quinolate), based on the sum weight of an inventive compound and the metal quinolate. In a further embodiment, the composition comprises from 20 to 50 weight percent of the metal quinolate (for example, lithium quinolate), based on the sum weight of an inventive compound and the metal quinolate.

The invention provides an article comprising at least one component formed from the composition of any one embodiment, or a combination of two or more embodiments, described herein. In one embodiment, the article is an organic electroluminescent device.

The invention provides a film comprising at least one layer formed from an inventive composition of any one embodiment, or a combination of two or more embodiments, described herein.

In one embodiment, the film further comprises a second layer, Layer B, formed from a Composition B, comprising at least one "HIL compound." In a further embodiment, the HIL (Hole Injection Layer) compound comprises an aromatic amine. In a further embodiment, the HIL compound is an aromatic diamine.

Examples of HIL compounds include, but are not limited to, 4,4',4"-tris(N,N-(2-naphthyl)-phenylamino)triphenylamine (2-TNATA); N1,N1'-([1,1'-biphenyl]-4,4'-diyl)bis(N1-(naphthalen-1 -yl)-N4,N4-diphenylbenzene-1 ,4-diamine); 4,4',4"-Tris[phenyl(m-tolyl)amino]triphenylamine (m-MTDATA); and N4,N4'-Bis[4-[bis(3-methylphenyl)amino]phenyl]-N4,N4'-diphenyl-[1,1'-biphenyl]-4,4'-diamine (DNTPD).

In one embodiment, Composition B comprises at least two HIL compounds. In a further embodiment, each HIL compound is independently a compound comprising an aromatic amine. In a further embodiment, each HIL compound

is independently an aromatic diamine.

In one embodiment, each film layer is formed by a vacuum deposition, thermal evaporation process.

In one embodiment, each film layer is formed by a solution process.

The invention also provides an article comprising at least one component formed from an inventive film. In a further embodiment, the article is an electroluminescent device.

The inventive compounds may be used as charge transporting layers and as other layers in electronic devices, such as OLED devices. For example, the inventive compounds may be used as charge blocking layers and charge generation layers

The invention provides an electronic device comprising at least one component formed from an inventive composition of any one embodiment, or a combination of two or more embodiments, described herein.

An inventive composition may comprise a combination of two or more embodiments described herein.

An inventive article may comprise a combination of two or more embodiments described herein.

An inventive film may comprise a combination of two or more embodiments described herein.

An inventive electronic device may comprise a combination of two or more embodiments described herein.

DEFINITIONS

The term "hydrocarbon," as used herein, refers to a chemical group containing only hydrogen and carbon atoms.

The term "substituted hydrocarbon," as used herein, refers to a hydrocarbon, in which at least one hydrogen atom is substituted with a heteroatom or a chemical group containing at least one heteroatom. Heteroatoms include, but are not limited to, O, N, P and S.

The term "aryl," *as* described herein, refers to an organic radical derived from aromatic hydrocarbon by deleting one hydrogen atom therefrom. An aryl group may be a monocyclic and/or fused ring system, each ring of which suitably contains from 4 to 7, preferably from 5 or 6 atoms. Structures wherein two or more aryl groups are combined through single bond(s) are also included. Specific examples include, but are not limited to, phenyl, naphthyl, biphenyl, anthryl, indenyl, fluorenyl, benzofluorenyl, phenanthryl, triphenylenyl, pyrenyl, perylenyl, chrysenyl, naphtacenyl, fluoranthenyl and the like, but are not restricted thereto. The naphthyl may be 1-naphthyl or 2-naphthyl, the anthryl may be 1-anthryl, 2-anthryl or 9-anthryl, and the fluorenyl may be any one of 1-fluorenyl, 2-fluorenyl, 3-fluorenyl, 4-fluorenyl and 9-fluorenyl.

The term "substituted aryl," as used herein, refers to an aryl, in which at least one hydrogen atom is substituted with a heteroatom or a chemical group containing at least one heteroatom. Heteroatoms include, but are not limited to, O, N, P and S.

The term "heteroaryl," as described herein, refers to an aryl group, in which at least one carbon atom or CH group or $CH_2$ is substituted with a heteroatom (for example, B, N, O, S, P(=O), Si and P) or a chemical group containing at least one heteroatom. The heteroaryl may be a 5- or 6-membered monocyclic heteroaryl or a polycyclic heteroaryl which is fused with one or more benzene ring(s), and may be partially saturated. The structures having one or more heteroaryl group(s) bonded through a single bond are also included. The heteroaryl groups may include divalent aryl groups of which the heteroatoms are oxidized or quarternized to form *N*-oxides, quaternary salts, or the like. Specific examples include, but are not limited to, monocyclic heteroaryl groups, such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups, such as benzofuranyl, fluoreno[4, 3-b]benzofuranyl, benzothiophenyl, fluoreno[4, 3-b]benzothiophenyl, isobenzofuranyl, benzimidazolyl, benzothiazolyl, benzisothiazolyl, benzisoxazolyl, benzoxazolyl, isoindolyl, indolyl, indazolyl, benzothia- diazolyl, quinolyl, isoquinolyl, cinnolinyl, quinazolinyl, quinoxalinyl, carbazolyl, phenanthridinyl and benzodioxolyl; and corresponding *N*-oxides (for example, pyridyl N-oxide, quinolyl N-oxide) and quaternary salts thereof.

The term "substituted heteroaryl," as used herein, refers to a heteroaryl, in which at least one hydrogen atom is substituted with a heteroatom or a chemical group containing at least one heteroatom. Heteroatoms include, but are not limited to, O, N, P and S.

The term "alkyl," *as* described herein, refers to an organic radical derived from an aliphatic hydrocarbon by deleting one hydrogen atom therefrom. An alkyl group may be linear, branched and/or cyclic. Specific examples include, but are not limited to, methyl, ethyl, propyl, tert-butyl, tert-octyl, pentyl, hexyl, cyclopropyl, cyclobutyl, cyclopentyl, cyclohexyl, etc..

The term "substituted alkyl," as used herein, refers to an alkyl, in which at least one hydrogen atom is substituted with a heteroatom or a chemical group containing at least one heteroatom. Heteroatoms include, but are not limited to, O, N, P and S.

The term "heteroalkyl," as described herein, refers to an alkyl group, in which at least one carbon atom or CH group or $CH_2$ is substituted with a heteroatom (for example, B, N, O, S, P(=O), Si and P) or a chemical group containing at least one heteroatom.

The term "substituted heteroalkyl," as used herein, refers to a heteroaryl in which at least one hydrogen atom

is substituted with a heteroatom or a chemical group containing at least one heteroatom. Heteroatoms include, but are not limited to, O, N, P and S.

EXPERIMENTAL

***Reagents and Test Methods***

All solvents and reagents were obtained from commercial vendors, including Sigma-Aldrich, Fisher Scientific, Acros, TCI and Alfa Aesar, and were used in the highest available purities, and/or were, when necessary, recrystallized before use. Dry solvents were obtained from in-house purification/dispensing system (hexane, toluene, tetrahydrofuran and diethyl ether), or purchased from Sigma-Aldrich. All experiments involving water sensitive compounds were conducted in "oven dried" glassware, under nitrogen atmosphere, or in a glovebox. Reactions were monitored by analytical thin-layer chromatography (TLC) on precoated aluminum plates (VWR 60 F254), visualized by UV light and/or potassium permanganate staining. Flash chromatography was performed on an ISCO COMBIFLASH system with GRACERESOLV cartridges.

1H-NMR-spectra (500 MHz or 400 MHz) were obtained on a Varian VNMRS-500 or VNMRS-400 spectrometer at 30°C, unless otherwise noted. The chemical shifts were referenced, depending on the NMR solvent used, to one of the following: TMS in $CHCl_3$ ($\delta$=0.00) in CDCl3, Benzene-$d_5$ (7.15) in Benzene-$d_6$ or DMSO-$d_5$ ($\delta$ 2.50) in DMSO-$d_6$. If necessary, peak assignment was carried out with the help of COSY, HSQC or NOESY experiments to confirm structural identity.

$^{13}$C-NMR spectra (125 MHz or 100 MHz) were obtained on a Varian VNMRS-500 or VNRMS-400 spectrometer, and referenced, depending on the NMR solvent used, to solvent or standard signals (0.0 - TMS in $CDCl_3$, 128.02 - Benzene-$d_6$, 39.43-DMSO-$d_6$).

Routine LC/MS studies were carried out as follows. Five microliter aliquots of the sample, as "3 mg/ml solution in THF," were injected on an AGILENT 1200SL binary gradient liquid chromatography, coupled to an AGILENT 6520 QT of, quadrupole-time of flight MS system, via a dual spray electrospray (ESI) interface operating in the PI mode. The following analysis conditions were used: column: 150 x 4.6 mm ID, 3.5 $\mu$m ZORBAX SB-C8; column temperature: 40°C; mobile phase: 75/25 A/B to 15/85 A/B at 40 minutes; solvent A = 0.1v% formic acid in water; solvent B = THF; flow 1.0 mL/min; UV detection: diode array 210 to 600 nm (extracted wavelength 250 nm, 280 nm); ESI conditions: gas temperature 365°C; gas flow - 8ml/min; capillary - 3.5 kV; nebulizer - 40PSI; fragmentor -145V.

DSC measurements were determined on a TA Instruments Q2000 instrument, at a scan rate of 10°C/min, and in a nitrogen atmosphere for all cycles. The sample was scanned from room temperature to 300°C, cooled to - 60°C, and reheated to 300°C. The glass transition temperature ($T_g$) was measured on the second heating scan. Data analysis was performed using TA Universal Analysis software. The $T_g$ was calculated using an "onset-at-inflection" methodology.

All computations utilized the Gaussian09 program[1]. The calculations were performed with the hybrid density functional theory (DFT) method, B3LYP,[2] and the 6-31G* (5d) basis set.[3] The singlet state calculations used the closed shell approximation, and the triplet state calculations used the open shell approximation. All values are quoted in electronvolts (eV). The HOMO and LUMO values were determined from the orbital energies of the optimized geometry of the singlet ground state. The triplet energies were determined as the difference between the total energy of the optimized triplet state and the optimized singlet state.

**1.** Gaussian 09, Revision A.02, Frisch, M.J. et al.; Gaussian, Inc., Wallingford CT, 2009. **2.** (a) Becke, A.D. J. Chem. Phys. 1993, 98, 5648. (b) Lee, C.; Yang, W.; Parr, R.G. Phys. Rev B 1988, 37, 785. (c) Miehlich, B.; Savin, A.; Stoll, H.; Preuss, H. Chem. Phys. Lett. 1989, 157, 200. **3.** (a) Ditchfield, R.; Hehre, W.J.; Pople, J.A. J. Chem. Phys. 1971, 54, 724. (b) Hehre, W.J.; Ditchfield, R.; Pople, J.A. J. Chem. Phys. 1972, 56, 2257. (c) Gordon, M.S. Chem. Phys. Lett. 1980, 76, 163.

The procedure described in the literature (J. Phys. Chem. A, 2003, 107, 5241-5251) was applied to calculate the reorganization energy ($\lambda^-$) of each molecule, which is an indicator of electron mobility.

Some embodiments of the invention are described in detail in the following examples.

*Synthetic Route for Inventive Compound **d***

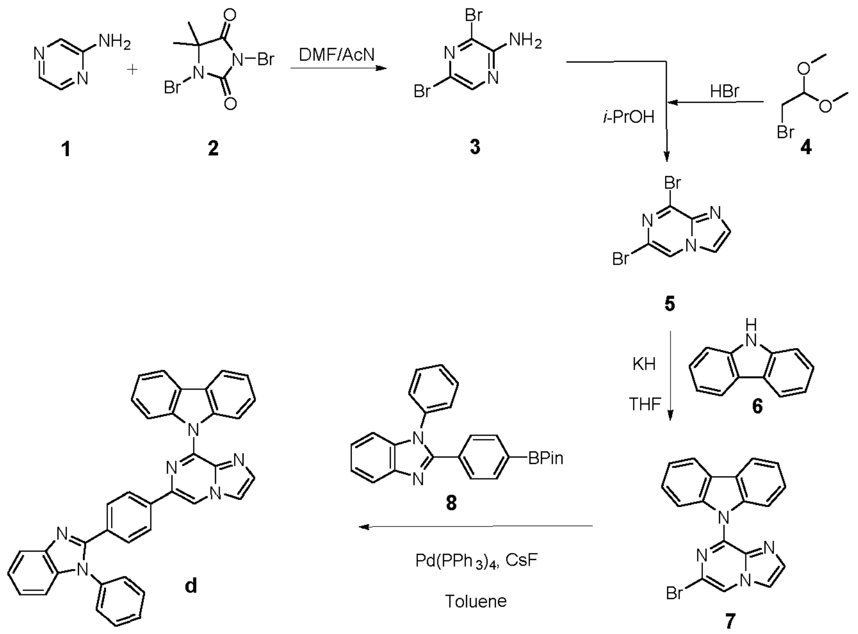


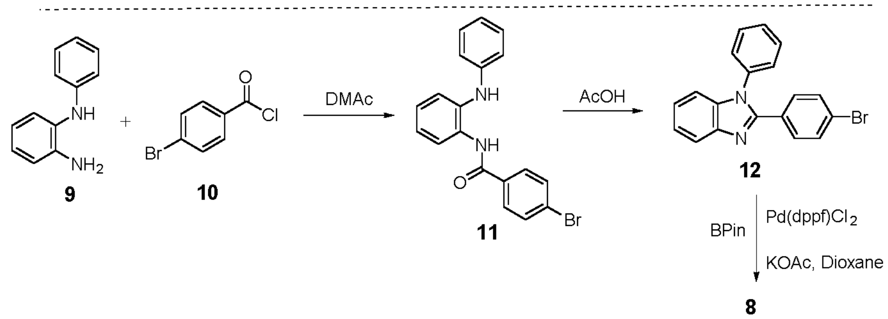


*Synthesis of intermediate 3,5-dibromopyrazin-2-amine (**3**)*

A solution of 2-aminopyrazine **1** (4.5 g, 47.4 mmol) in 50 mL DMF/MeCN solvent mixture (1:3) and a solution of 1,3-dibromo-5,5-dimethylhydantoin **2** (13.6 g, 47.6 mmol, 2 eq. of "Br") in 40 mL DMF/MeCN solvent mixture (1:3) were simultaneously added (using two syringe needles) to a solution of MeCN (100 mL) at 0°C. The reaction mixture was stirred for one hour, quenched with aqueous sodium thiosulfate (1N, 200 mL) and concentrated under vacuum. The crude product (>99% conversion, 8 g) was dissolved in EtOAc and filtered through celite/charcoal and recrystallized from acetonitrile (4.0 g, 50% recovery). The reaction was scaled up to 12.0 g in two batches and the desired product **3** was analyzed by NMR and LC-MS. 1H NMR (500 MHz, CDCl3) δ 7.99 (s, 1H); 13C NMR (126 MHz, CDCl3) δ 151.92, 143.16, 142.99, 123.95, 123.56.

*Synthesis of intermediate 6,8-dibromoimidazo[1,2-a]pyrazine (**5**)*

A 100-mL, three-neck flask was charged with 2-bromo-1,1-dimethoxyethane **4** (23.2 g (16 mL, 137.2 mmol) and aqueous HBr (47.6% HBr by wt, 8.8 mmol/mL, 6.6 mL, 58.8 mmol), and the reaction mixture was heated to reflux (55°C) for 2 hours. The mixture was allowed to cool to 40°C, and solid $NaHCO_3$ was added in small portions, until evolution of gas ceased. The resulting suspension was filtered, under vacuum, into a 500-mL, three-neck flask, and the filter cake was washed with isopropanol (200 mL). Solid 3,5-dibromopyrazin-2-amine (**3**) (12.0 g, 47.2 mmol) was added into the isopropanol filtrate, and the reaction mixture was heated at reflux (78°C) for 16 hours. The resulting suspension was cooled to room temperature, filtered, the cake was washed with cold isopropanol (100 mL), and then the cake was dried under vacuum. The cake was transferred into a three-neck flask, into which, water (200 mL) was added, followed by solid $K_2CO_3$, in small portions, until gas evolution ceased, after which the reaction was stirred for 30 minutes. The resulting precipitate was isolated by filtration, and washed with water (200 mL). The solid was dried at 50°C, to constant weight, then dissolved in THF, filtered through a plug (celite, silica gel, and charcoal). The solvent was removed under vacuum, to give 12.0 g of the desired product (**5**) as a white solid at 99% purity, as determined by NMR and LC-MS. 1H

NMR (500 MHz, CDCl3) δ 8.26 (s, 1H), 7.84 (d, J = 1.0 Hz, 1H), 7.78 (d, J = 1.1 Hz, 1H); 13C NMR (126 MHz, CDCl3) δ 136.93, 134.06, 120.29, 119.26, 115.78.

*Synthesis of intermediate 9-(6-bromoimidazo[1,2-a]pyrazin-8-yl)-9H-carbazole (7)*

Potassium hydride (washed with hexanes and dried) (145 mg, 3.6 mmol) was added to a solution of carbazole **6** (302 mg, 1.8 mmol), in THF (5 mL), at 0°C. The reaction mixture was stirred for 20 minutes (solution turns greenish), after which, this solution was added dropwise to a solution of 6,8-dibromoimidazo[1,2-a]pyrazine (2) (500 mg, 1.8 mmol), in THF (5 mL) (reaction mixture turns reddish-brown). The reaction mixture was allowed to warm to room temperature, and stirred for 2 hours; the time during which an aliquot was analyzed by LC-MS to check reaction progress. After the reaction had gone to completion (>90% conversion), the solvent was removed under vacuum, and the residue dissolved in hot chloroform, from which it was recrystallized, to give approximately 0.5 g (76% yield) of the desired product (**7**), at approximately 98% purity by LC-MS and 1H-NMR. The reaction was successfully scaled up to 4 g. 1H NMR (500 MHz, CDCl3) δ 8.32 (s, 1H), 8.12 - 8.08 (m, 2H), 7.85 (d, J = 1.0 Hz, 1H); 7.79 (d, J = 1.0 Hz, 1H), 7.76 (s, 1H), 7.74 (s, 1H), 7.44 (ddd, J = 8.4, 7.2, 1.3 Hz, 2H), 7.38 - 7.33 (m, 2H).

*Synthesis of intermediate 4-bromo-N-(2-(phenylamino)phenyl)benzamide* (**11**)

A 1-L round bottom flask was charged with 4-bromobenzoylchloride **13** (24.0 g, 110 mmol) and *N,N*-dimethylacetamide (DMAc, 200 mL). A solution of *N*-phenylbenzene-1,2-diamine **12** (20.0 g, 109 mmol) in DMAc (300 mL) was added dropwise into the flask over 30 min using an addition funnel. Upon complete addition (>95% conversion), the reaction solution was stirred overnight (14 h) at room temperature. The dark colored reaction mixture was transferred into a 250-mL Erlenmyer flask into which was added 200 mL of deionized water causing a precipitate to form (color changes to red). This mixture was stirred for 1 h at room temperature then filtered over a course porosity fritted filter. The filtrate was washed with hexanes (3 x 200 mL) and dried in a vacuum oven (60 °C) for 24 h to give 36.0 g (90% yield) of the desired amide **14** in ~98% purity as determined by NMR and LC-MS (Waters e2695 system equipped with a Waters 2424 ELS Detector, Waters 2998 Photodiode Array Detector, and a 3100 mass detector in positive ion mode). $^1$H NMR (500 MHz, $CDCl_3$) δ 8.36 (s, 1H), 8.23 (d, *J* = 7.1 Hz, 1H), 7.54 - 7.45 (m, 4H),7.27 - 7.21 (m, 4H), 7.19 - 7.13 (m, 1H), 6.91 (tt, *J* = 7.5, 1.0 Hz, 1H), 6.80 (dt, *J* = 8.8, 1.6 Hz, 2H), 5.62 (s, 1H); $^{13}$C NMR (126 MHz, $CDCl_3$) δ 164.62, 144.72, 133.50, 132.87, 132.74, 131.91, 129.55, 128.58, 126.53, 125.57, 125.48, 124.70, 121.93, 120.62, 116.07.

*Synthesis of intermediate 2-(4-bromophenyl)-1-phenyl-1H-benzo[d]imidazole* (**12**)

A 1-L three-neck round bottom flask was charged with 4-bromo-*N*-(2-(phenylamino)-phenyl)benzamide **11** (30.0 g, 82.0 mmol) followed by glacial acetic acid (300 mL). The mixture was refluxed (100 °C) with stirring for 12 h, after which LC-MS of an aliquot showed 95% conversion. Acetic acid was removed under reduced pressure and the residue was triturated with hot hexanes to give a purple solid (34.0 g) after removing solvent under reduced pressure. The crude solids were dissolved in a minimal amount of chloroform and separated into two equal portions. Each portion was passed through slurry of silica gel topped with sea sand. The plug was washed with chloroform enough times to elute the desired compound. The solvent was removed under reduced pressure and the product recrystallized from chloroform/hexanes to give a white solid (24.0 g, 84% yield) of the desired compound **12** at ~98% purity by LC-MS and NMR. $^1$H NMR (500 MHz, $CDCl_3$) δ 7.89 (d, *J* = 8.0 Hz, 1H), 7.56 - 7.48 (m, 3H), 7.48 - 7.41 (m, 4H), 7.38 - 7.33 (m, 1H), 7.33 - 7.27 (m, 3H), 7.27 - 7.22 (m, 1H); $^{13}$C NMR (126 MHz, $CDCl_3$) δ 151.17, 142.90, 137.24, 136.74, 131.51, 130.80, 129.96, 128.89, 128.73, 127.33, 124.00, 123.54, 123.11, 119.88, 110.43.

*Synthesis of intermediate 1-phenyl-2-(4-(4,4,5,5-tetramethyl-1,3,2-dioxaborolan-2-yl)-phenyl)-1H-benzo[d]imidazole* (**8**)

In a glovebox, three oven-dried reaction jars were each charged with 2-(4-bromophenyl)-1-phenyl-1H-benzimidazole **12** (4.20 g, 12.0 mmol), bis(pinacolato)-diboron (Bpin) (3.05 g, 12 mmol, 1 eq.), potassium acetate (2.95 g, 30 mmol, 2.5 eq.), and Pd(dppf)$Cl_2$ (300 mg, 0.36 mmol, 3 mol%). The solvent (1,4-dio-xane, 80 mL) was added to each jar and the reactions were stirred at 80 °C overnight. After complete conversion (~95%) as determined by LC-MS, the contents of the reaction jars were mixed and the solvent removed under reduced pressure. Water (200 mL) was added the product was extracted into chloroform (3x200 mL), the product was passed through a silica plug and recrystallized from acetonitrile. The reaction was successfully scaled up to 12 g in 90% yield after recrystallization to give the desired boronic ester **8** at ~98% purity as judged by LC-MS and NMR. $^1$H NMR (500 MHz, $cdcl_3$) δ 7.90 (dt, *J* = 8.1, 0.9 Hz, 1H), 7.76 - 7.71 (m, 2H), 7.60 - 7.55 (m, 2H), 7.49 - 7.41 (m, 3H), 7.35 - 7.21 (m, 6H), 1.32 (s, 12H); $^{13}$C NMR (126 MHz, $CDCl_3$) δ 172.37, 157.10, 152.56, 135.25, 133.58, 133.11, 131.25, 130.46, 128.49, 128.13, 128.03, 127.96, 127.73, 127.11, 126.47, 125.24, 124.99, 123.30

*Synthesis of Inventive compound **d***

To a solution of 9-(6-bromoimidazo[1,2-a]pyrazin-8-yl)-9H-carbazole (7) (3.3 g, 9.2 mmol), in toluene/water (80 mL), 1-phenyl-2-(4-(4,4,5,5-tetramethyl-1,3,2-dioxaborolan-2-yl)phenyl)-1Hbenzo[d]imidazole (**8**) (10.1 mmol, 1.1 eq.), CsF (4.20 g, 27.6 mmol, 3 eq.), and $Pd(PPh_3)_4$ were added. The reaction mixture was stirred overnight at 90°C, after which, LC-MS analysis of an aliquot of the reaction mixture showed >95% conversion of starting material to desired product. The reaction mixture was allowed to cool to room temperature, chilled in ice, and the solid product collected by filtration. The product was washed with hexanes, and dried, to give 4.5 g (88% yield; approx. 97% pure) of the desired product, as determined by LCMS and 1H NMR. The product was washed with boiling water to remove salts, and washed with ethyl acetate and methanol to remove organic impurities, and then recrystallized from refluxing chlorobenzene to remove color and residual impurities, and finally filtered, under vacuum, to give 2.5 g of the desired compound (**d**), at 99.6% purity, as determined by 1H-NMR and LC-MS. 1H NMR (500 MHz, CDCl3) δ 8.59 (s, 1H), 8.12 (ddd, J = 7.7, 1.2, 0.6 Hz, 2H), 8.00 - 7.95 (m, 2H),7.94 (dt, J = 8.1, 0.9 Hz, 1H), 7.84 (s, 2H), 7.76 -7.69 (m, 4H), 7.56 - 7.46 (m, 3H), 7.45 - 7.27 (m, 9H); 13C NMR (126 MHz, CDCl3) δ 151.67, 143.06, 142.87, 139.85, 137.39, 137.04, 136.91, 136.55, 135.56, 135.41, 130.43, 130.02, 128.76, 127.42, 125.94, 125.84, 125.07, 123.55, 123.14, 121.50, 120.02, 119.90, 115.45, 113.92, 113.08, 110.53.

*Synthetic Route for Inventive Compound **c***

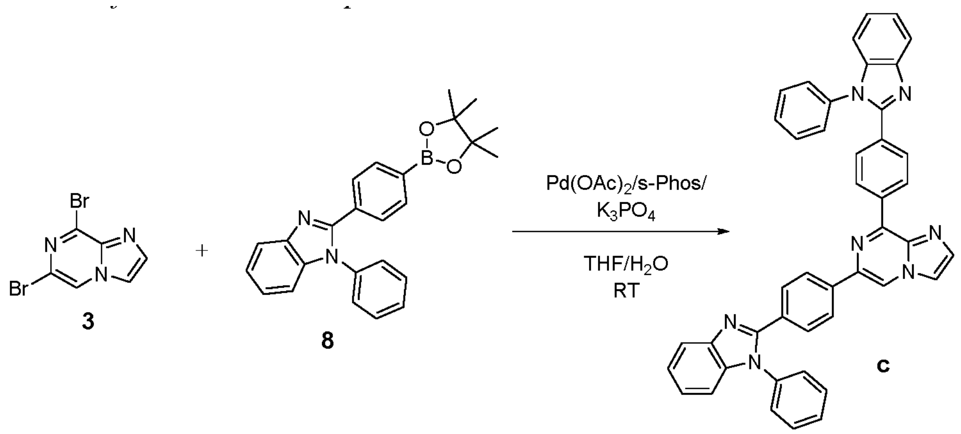


3

8

c

In a glovebox, a reaction vial was charged with 6,8-dibromoimidazo[1,2-a]pyrazine (**3**) (5.54 g, 20 mmol), 1-phenyl-2-(4-(4,4,5,5-tetramethyl-1,3,2-dioxaborolan-2-yl)phenyl)-1H-benzo[d]imidazole (**8**) (15.6 g, 40 mmol, 2 equiv.), $K_3PO_4$ (12.7 g, 60 mmol, 3 equiv.), s-Phos (41 mg, 0.1 mmol, 0.5 mol %), and $Pd(OAc)_2$ (22.5 mg, 0.1 mmol, 0.5 mol%). THF (80 mL) and water (8 mL) were added, and the mixture was vigorously stirred at room temperature. A thick, whitish precipitate formed over time; the reaction progress was monitored by LC-MS, until all dibromoimidazoprazine **3** was consumed. The solvent was removed by rotary evaporation, and the solid was re-dissolved in chloroform, washed with water, dried, and purified by flash column chromatography, to give 8 g (61%) of the desired compound (**c**). 1H NMR (400 MHz, Chloroform-d) δ 8.88 - 8.76 (m, 2H), 8.01- 7.84 (m, 4H), 7.83 - 7.71 (m, 3H), 7.72-7.61 (m, 3H), 7.56 - 7.40 (m, 6H), 7.39 - 7.28 (m, 5H), 7.29 -7.16 (m, 4H). 13C NMR (101 MHz, Chloroform-d) δ 151.85, 151.73, 147.52, 143.02, 142.94, 138.64, 138.04, 137.71, 137.38, 137.29, 137.21, 136.93, 136.91, 136.69, 135.38, 131.67, 130.06, 129.97, 129.94, 129.87, 129.53, 129.31, 128.68, 127.44, 127.40, 125.87, 123.52, 123.47, 123.08, 119.90, 119.78, 114.54, 114.44, 110.50, 110.47.

*Synthetic Route for Inventive Compound **a***

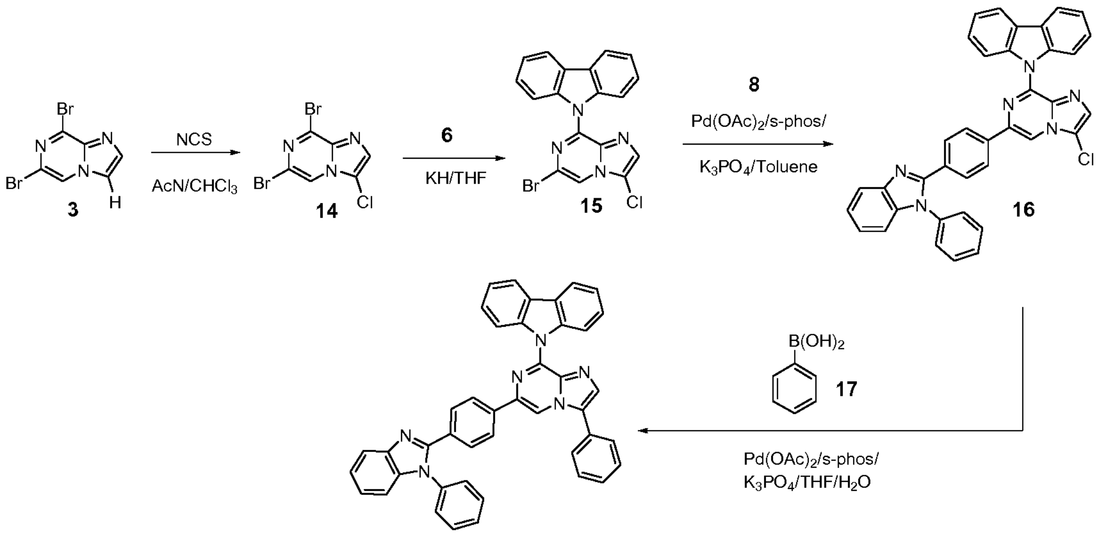


3 14 15 16 a

*Synthesis of intermediate of 6,8-dibromo-3-chloroimidazo[1,2-a]pyrazine (**14**)*

The 3,5-dibromoimidazopyrazine 3 (3.9 g, 14 mmol) was charged into a 250 mL round bottom flask (immersed in ice/water), chloroform (40 mL) and acetonitrile (40 mL) followed by a solution of *N*-chlorosuccinimide **2** (2.8 g, 21 mmol, 1.5 equiv) in $CHCl_3$/MeCN (1:2) (1 mL). The reaction was allowed to warm to room temperature and then heated at 50 °C with a reflux condenser and monitored by LC-MS until complete conversion to the desired product **14.** $^1$H NMR (500 MHz, Chloroform-*d*) δ 8.20 - 8.11 (m, ls), 7.79 - 7.70 (m, ls). $^{13}$C NMR (126 MHz, $cdcl_3$) δ 134.16, 133.91, 133.88, 121.30, 120.93, 109.99.

*Synthesis of intermediate 9-(6-bromo-3-chloroimidazo[1,2-a]pyrazin-8-yl)-9H-carbazole (**15**)*

Potassium hydride (KH) (washed with hexanes and dried) (640 mg, 16 mmol) was added to a solution of carbazole **6** (2.68g, 16 mmol, equiv), in THF (50 mL), at < 0°C, The reaction mixture was stirred for 10 minutes (solution turns greenish), after which, this solution was added dropwise to a solution of 6,8-dibromo-3-chloroimidazo[1,2-a]pyrazine (**14**) (5 g, 16 mmol) in THF (30 mL) (solution turns purple-brown). The reaction mixture was allowed to warm to room temperature, overnight, while stirring. Dry silica gel was added to the crude reaction mixture, and dried by rotary evaporation. The dry powder was loaded onto a cartridge, and purified by a TELEDYNE ISCO purification unit, to give the intermediate **(15)** (4 g, 63%). 1H NMR (400 MHz, Chloroform-d) δ 8.24 (s, 1H), 8.07 (ddd, J = 7.7, 1.4, 0.7 Hz, 2H), 7.74 - 7.70 (m, 2H), 7.41 (ddd, J = 8.4, 7.2, 1.3 Hz, 2H), 7.34 (ddd, J = 7.7, 7.2, 1.0 Hz, 2H). 13C NMR (101 MHz, Chloroform-d) δ 142.34, 139.40, 139.38, 135.93, 133.90, 132.68, 126.16, 125.39, 125.38, 122.23, 122.21, 121.40, 120.01, 115.61, 114.49, 113.28, 113.26.

*Synthesis of intermediate 9-(3-chloro-6-(4-(1-phenyl-1H-benzo[d]imidazol-2-yl)phenyl)imidazo[1,2-a]pyrazin-8-yl)-9H-carbazole **(16)***

In a glovebox, a reaction jar was charged with 9-(6-bromo-3-chloroimidazo[1,2-a]pyrazin-8-yl)-9Hcarbazole **(15)** (3 g, 7.5 mmol), 4-(1-phenyl-1H-benzo[d]imidazol-2-yl)phenylpinacol boronic ester **(8)** (2.97 g, 7.5 mmol, 1 equiv.), $K_2CO_3$ (3.1 g, 22.5 mmol, 3 equiv.), $PPh_3$ (196 mg, 0.75 mol, 10 mol %), and $Pd(OAc)_2$ (84 mg, 0.38 mmol, 5 mol%). Dry toluene (50 mL) was added, followed by nitrogen-sparged acetonitrile (10 mL). The reaction jar was then capped, tapped, and transferred to a "wet" glovebox, were nitrogen-sparged water (12 mL) was added, and the reaction was stirred overnight at 80°C. The reaction mixture changed from clear yellow solution to cream precipitate, after overnight reaction. The solvent was removed under vacuum, and the product re-dissolved in chloroform, and washed with water and brine. The organic fraction was dried with sodium sulfate, and the solvent removed by rotatory evaporation. To remove organics from the starting material and the catalyst, ethylacetate and methanol were added into the solid, and the mixture was heated to reflux for one hour, filtered, washed with copious amounts of water, and dried to give grey solids of intermediate (**16**) (4g, 91% yield, and ~99% purity by LC-MS). 1H NMR (400 MHz, Chloroform-d) δ 8.49 (s, 1H), 8.10 (ddd, J = 7.7, 1.4, 0.7 Hz, 2H), 8.07 - 7.99 (m, 2H), 7.91 (dt, J = 8.0, 0.9 Hz, 1H), 7.76 (s, 1H), 7.75 - 7.67 (m, 4H), 7.55 - 7.44 (m, 3H), 7.43 - 7.25 (m, 9H). 13C NMR (101 MHz, cdcl3) δ 151.50, 142.85, 139.70, 137.94, 137.33, 136.86, 136.26, 134.26, 132.33, 130.67, 130.07, 130.01, 128.79, 127.41, 126.03, 125.99, 125.14, 123.60, 123.18,

121.70, 120.05, 119.87, 113.45, 113.01, 110.50, 110.31.

*Synthesis of Inventive compound **a***

In a glovebox, a reaction vial was charged with 9-(3-chloro-6-(4-(1-phenyl-1H-benzo-[d]imidazol-2-yl)phenyl)imidazopyrazin-8-yl)-9H-carbazole (**16**) (4 g, 6.8 mmol), phenyl-boronic acid (**17**) (1.2 g, 10.2 mmol, 1.5 equiv.), $K_3PO_4$ (3 g, 13.6 mmol, 2 equiv.), s-Phos (70 mg, 0.17 mmol, 2.5 mol %), and $Pd(OAc)_2$ (38 mg, 0.17 mmol, 2.5 mol%). THF (80 mL) and water (8 mL) were added, and the mixture was stirred overnight at 55°C. Crude product was dried by solvent evaporation on a rotorvap, and the resulting solid was triturated from boiling chlorobenzene, which removed most of the color and small impurities. The solid was filtered from hot chlorobenzene, and further triturated from a mixture of hot methanol and ethylacetate, further removing residual impurities and color. The solid was dissolved in chloroform, and silica gel was added, the solvent removed under vacuum, and the fine powder was loaded into a cartridge for column chromatography, using a TELEDYNE ISCO system. The product was eluted (120g column) using 0-10% EtOAc in chloroform. 1H NMR (400 MHz, Chloroform-d) δ 8.12 (ddd, J = 7.7, 1.3, 0.7 Hz, 2H), 8.00 - 7.86(m, 4H), 7.78 (dt, J = 8.3, 0.8 Hz, 2H), 7.73 - 7.58 (m, 6H), 7.59 - 7.39 (m, 6H), 7.39 - 7.30 (m, 5H), 7.31- 7.18 (m, 2H). 13C NMR (101 MHz, Chloroform-d) δ 139.90, 129.99, 129.70, 129.38, 128.73, 128.33, 127.40, 125.94, 125.93, 125.07, 123.13, 121.49, 120.04, 119.86, 113.10, 110.49.

*Synthetic Route for Inventive compound **b***

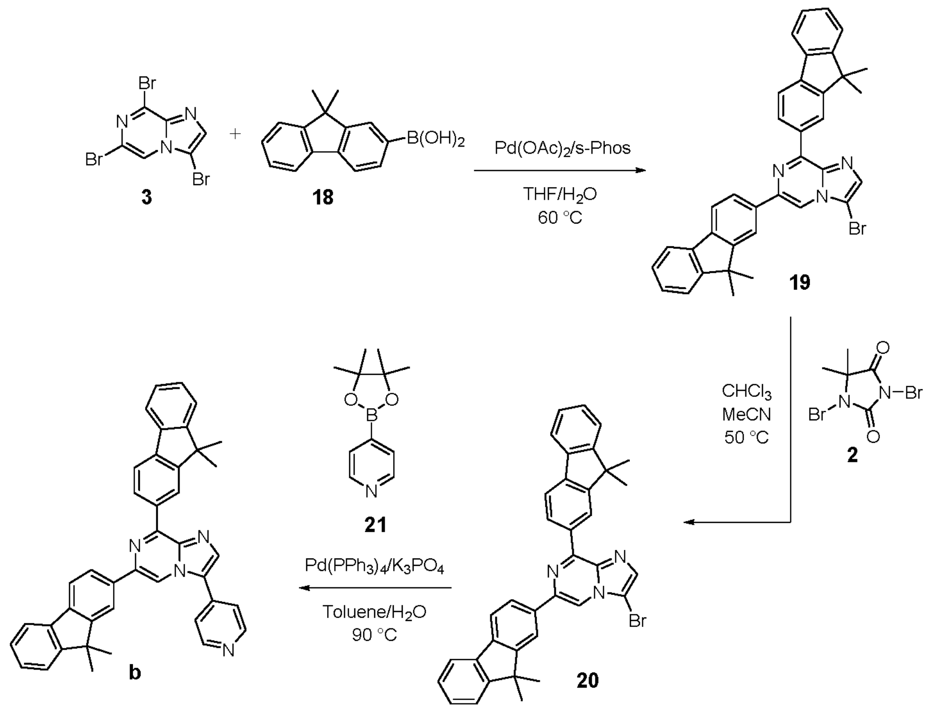


*Synthesis of intermediate 6,8-bis(9,9-dimethyl-9H-fluoren-2-yl)imidazo[1,2-a]pyrazine (**19**)*

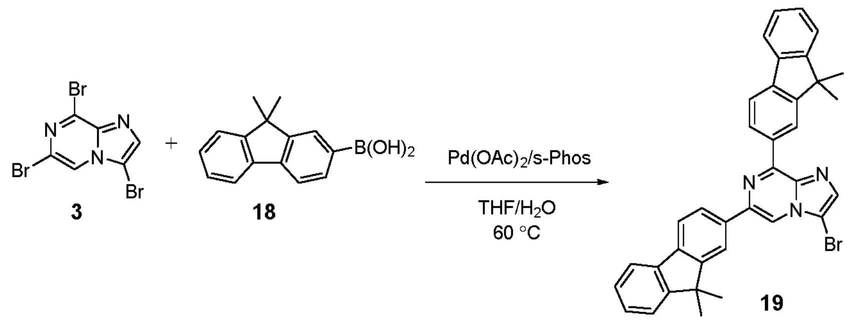


In a glovebox, a reaction vial was charged with 6,8-dibromoimidazo[1,2-a]pyrazine (**3**) (4.8 g, 17.2 mmol), 2-(9,9-dimethyl-9H-fluoren-2-yl)-4,4,5,5-tetramethyl-1,3,2-dioxaborolane (**18**) (8.2 g, 34.5 mmol, 2 equiv.), $K_3PO_4$ (10.9 g, 51.6 mmol, 3 equiv.), s-Phos (356 mg, 0.86 mmol, 5 mol %), and $Pd(OAc)_2$ (193 mg, 0.86 mmol, 5 mol%). THF (150 mL) and water (15 mL) were added, and the mixture was stirred at 60°C overnight. An aliquot of the reaction mixture was analyzed by LC-MS, and found to have approx. 10% starting material remaining. More dimethylfluorenylboronic acid **18** (300 mg) and $K_3PO_4$ (100 mg) were added into the reaction mixture, and the reaction was stirred overnight at 60°C. The solvent was removed by rotatory evaporation, and the resulting solid (7.8 g) was dissolved in chloroform, washed with water and brine, the organic fraction was dried by sodium sulfate, and the solvent removed by rotatory evaporation. The resulting solid was washed, triturated with methanol, and the solids filtered, and dried to give a yellowish solid intermediate (**19**) (5g, 58% yield), which was analyzed by LC-MS (98% pure). 1H NMR (400 MHz, Chloroform-d) δ 9.03 -8.94 (m, 2H), 8.48 (s, 1H), 8.17 (dd, J = 1.7, 0.7 Hz, 1H), 8.06 (dd, J = 7.9, 1.7 Hz, 1H), 7.94 (dd, J = 7.8, 0.9 Hz, 1H), 7.90 - 7.81 (m, 3H), 7.81 - 7.75 (m, 2H), 7.53 - 7.45 (m, 2H), 7.43 - 7.31 (m, 4H), 1.62 (d, J= 14.8 Hz, 12H). 13C NMR (101 MHz, Chloroform-d) δ 154.59, 154.32, 154.02, 153.60, 149.01, 141.46, 139.83, 139.27, 138.96, 138.79, 138.69, 135.88, 135.28, 135.20, 129.29, 127.77, 127.54, 127.10, 127.05, 125.41, 124.01, 122.71, 122.68, 120.61, 120.40, 120.25, 119.80, 114.20, 113.58, 47.11, 27.26, 27.25.

*Synthesis of intermediate 3-bromo-6,8-bis(9,9-dimethyl-9H-fluoren-2-yl)imidazo[1,2-a]pyrazine (**20**)*

Bisdimethylfluorenylimidazopyrazine (**19**) (2 g, 4 mmol) was charged into a reaction vial, into which, dry chloroform (50 mL) and dry acetonitrile (50 mL) were added, followed by a solution of 5,5-dimethyl-1,3-dibromohydantoin **2** (0.59 g, 2.1 mmol, 1.1 'Br' equiv) in dry CHCl3/MeCN (1:2) (9 mL) slowly (reaction turns greenish). The reaction was stirred at room temperature, and the reaction progress was monitored, by taking an aliquot of the reaction mixture for LC-MS analysis, until complete conversion to the desired product (approx. one hour). Then, 3.2 g of crude product was dissolved in chloroform, silica gel was added, and the solvent was removed by rotatory evaporation, until a free-flowing powder was obtained. The solid was loaded onto a cartridge, and the product was eluted with 80% chloroform in hexanes. The product was combined, solvent evaporated, and the purity of the final product **20** (2.2 g, 94% yield) was analyzed by LC-MS. 1H NMR (400 MHz, Chloroformd) δ 8.96 - 8.87 (m, 2H), 8.43 (s, 1H), 8.20 (dd, J = 1.7, 0.6 Hz, 1H), 8.10 (dd, J = 7.9, 1.7 Hz, 1H), 7.92 (d, J = 8.4 Hz, 1H), 7.90 - 7.84 (m, 2H), 7.84 - 7.77 (m, 2H), 7.53 - 7.45 (m, 2H), 7.42 - 7.33 (m, 4H), 1.62 (d, J = 5.7 Hz, 12H). 13C NMR (101 MHz, Chloroform-d) δ 154.58, 154.39, 154.08, 153.65, 149.02, 141.73, 140.34, 140.13, 138.67, 138.60, 135.65, 135.25, 134.60, 129.34, 127.87, 127.62, 127.10, 127.07, 125.64, 124.11, 122.71, 122.69, 120.85, 120.64, 120.43, 120.30, 119.85, 111.02, 97.82, 47.11, 47.06, 27.24, 27.21.

*Synthesis of compound **b***

In a glovebox, a reaction jar was charged with bromo-bis(dimethylfluorenyl)-imidazopyrazine (**20**) (3.2 g, 5.5 mmol), pyridine-4-pinacolboronic ester **21** (1.36 g, 6.6 mmol, 1.2 equiv), $K_3PO_4$ (3.5 g, 16.5 mmol, 3 equiv), $Pd(PPh_3)_4$ (318 mg, 0.275 mmol, 5 mol%), followed by toluene (100 mL) and water (20 mL). The reaction was stirred at 90°C over the weekend. An aliquot of the reaction mixture indicated approx. 70% conversion to the desired product. Additional $K_3PO_4$ (0.9 g), and $Pd(PPh_3)_4$ (32 mg) were added, and the reaction was stirred overnight at 90°C, after which, an aliquot indicated complete conversion to the desired product. The crude product was washed with water and brine, dried with silica gel, and solid-loaded onto an ISCO purification unit. The product was eluted with 100% EtOAc, and the fractions containing the desired product were combined, and the solvent removed by rotatory evaporation. The solid (2.9 g, 94% yield) was triturated with methanol, to give a yellow solid (2.2 g) that was analyzed for purity by LC-MS (99.38% pure). 1H NMR (400 MHz, Chloroform-d) δ 8.98 - 8.89 (m, 2H), 8.89 - 8.80 (m, 2H), 8.64 (s, 1H), 8.15 (dd, J = 1.7, 0.6 Hz, 1H), 8.05 (s, 1H), 7.98 (dd, J = 7.9, 1.7 Hz, 1H), 7.94 (d, J = 8.4 Hz, 1H), 7.89 - 7.80 (m, 2H), 7.80 - 7.74 (m, 1H), 7.66 - 7.58 (m, 2H), 7.54 - 7.43 (m, 2H), 7.43 - 7.31 (m, 4H), 1.60 (d, J = 17.6 Hz, 12H). 13C NMR (101 MHz,

Chloroform-d) δ 214.28, 154.58, 154.45, 154.05, 153.65, 151.05, 150.00, 141.74, 140.77, 140.16, 140.12, 138.66, 138.52, 136.31, 135.72, 135.56, 134.99, 129.35, 127.89, 127.67, 127.11, 127.08, 125.51, 124.71, 124.13, 122.71, 122.69, 121.77, 120.97, 120.64, 120.41, 120.30, 119.85, 110.56, 47.12, 47.07, 27.22.

Each inventive compound can be used to form film layers for an electronic device, such as an organic electroluminescense device.

***Device Experimental***

***Definition***

A Conversion efficiency is defined as the value that is luminance efficiency divided by CIE Y. Especially, luminance efficiency depending on color coordinates in blue device is highly considered factor.

$$\text{Conversion efficiency} = \frac{\text{Luminance efficiency}}{\text{CIE Y}}$$

***OLED Device Fabrication and Testing***

All organic materials were purified by sublimation before deposition. OLEDs were fabricated onto an ITO coated glass substrate that served as the anode, and topped with an aluminum cathode. All organic layers were thermally deposited by physical vapor deposition, in a vacuum chamber with a base pressure of $<10^{-7}$ torr.

Each cell, containing HIL, HTL, EML host, EML dopant, ETL, or EIL, was placed inside a vacuum chamber, until it reached $10^{-6}$ torr. To evaporate each material, a controlled current was applied to the cell, containing the material, to raise the temperature of the cell. An adequate temperature was applied to keep the evaporation rate of the materials constant throughout the evaporation process.

For the HIL layer, *N4,N4'-diphenyl-N4,N4'-bis(9-phenyl-9H-carbazol-3-yl)-[1,1'-biphenyl]-4,4'-diamine* was evaporated, until the thickness of the layer reached 60nm. For the HTL layer, *N-([1,1'-biphenyl]-4-yl)-9,9-dimethyl-N-(4-(9-phenyl-9H-carbazol-3-yl)phenyl)-9H-fluoren-2-amine* was evaporated, until the thickness reached 25nm. For the EML layer, 9-*phenyl-10-(4-phenylnaphthalen-1-yl)anthracene* (BH-1, host) and *Nl,N6-bis(5'-fluoro-[1,1':3',1"-terphenyl]-4'-yl)-N1,N6-diphenylpyrene-1,6-diamine* (BD-1, dopant) were co-evaporated, until the thickness reached 20 nm. The doping ratio for the dopant material was 2wt%. For the ETL layer, the ETL compounds were co-evaporated with lithium quinolate (Liq), until the thickness reached 30nm with evaporation ratio of 1:1.Alq3 (*tris(8-hydroxyquinolinato)aluminum)* was used as a reference material to compare with the inventive compounds. Alq3 was evaporated solely at 1A/s rate, until 30nm. Finally, "2nm" of a thin electron injection layer (Liq) was evaporated. See Table 1.

The current density-voltage-luminance (J-V-L) characterizations for the OLED devices were performed with a source measurement unit (KEITHLY 2635A) and a luminescence meter (MINOLTA CS-100A). EL spectra of the OLED devices were collected by a calibrated CCD spectrograph. See Table 2.

Table 1: Device Materials

| Material | Name |
|---|---|
| Hole Injection Material | *N4,N4'-diphenyl-N4,N4'-bis(9-phenyl-9H-carbazol-3-yl)-[1,1'-biphenyl]-4,4'-diamine*<br>CAS No. 887402-92-8 |
| Hole Transporting Material | *N-([1,1'-biphenyl]-4-yl)-9,9-dimethyl-N-(4-(9-phenyl-9H-carbazol-3-yl)phenyl)-9H-fluoren-2-amine*<br>CAS No. 1242056-42-3 |
| Fl Blue Host (BH-1) | *9-phenyl-10-(4-phenylnaphthalen-1-yl)anthracene*<br>CAS No. 1207336-74-0 |
| Fl Blue Dopant (BD-1) | *N1,N6-bis(5'-fluoro-[1,1':3',1"-terphenyl]-4'-yl)-N1,N6-diphenylpyrene-1,6-diamine*<br>CAS No. 1228525-73-2 |
| Ref ETL | *Tris(8-hydroxyquinolinato)aluminum*<br>CAS N. 2085-33-8 |

(continued)

| Material | Name |
|---|---|
| Electron Injection Material | *Lithium quinolate (Liq)*<br>CAS No. 850918-68-2 |
| Weight ratio of each ETL:Liq is 50:50 | |

Table 2: Device results

| ETL | Voltage @1000nit/ @50mA/cm$^2$ (V) | Conversion Efficiency [Luminance efficiency/CIE Y] @ 1000nit | CIE (X,Y) @1000nit |
|---|---|---|---|
| Ref ETL | 6.5 / 7.3 | 37.5 | 0.142, 0.104 |
| Compound (a):Liq | 6.2 / 7.2 | 52.6 | 0.139, 0.095 |
| Compound (b):Liq | 5.8 / 6.5 | 44.2 | 0.139, 0.095 |
| Compound (c):Liq | 6.9 / 7.3 | 46.2 | 0.139, 0.093 |

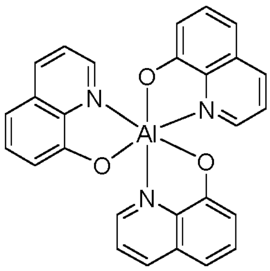
Ref ETL ,
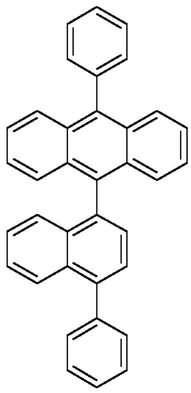
BH-1 ,
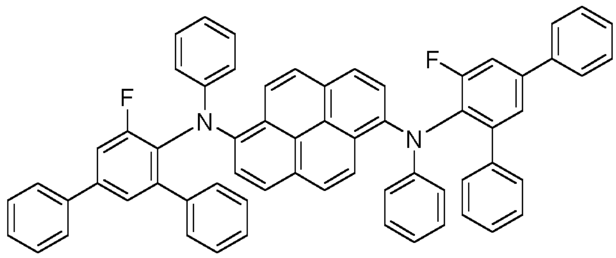
BD-1

**Claims**

**1.** A composition comprising at least one compound selected from the following (a) through (v1):

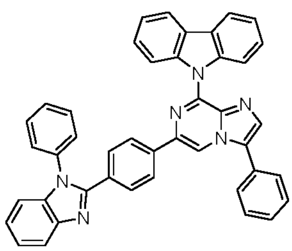
(a),
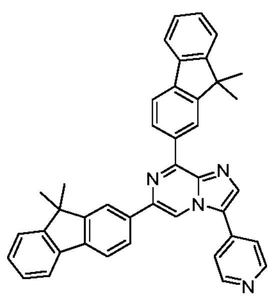
(b),
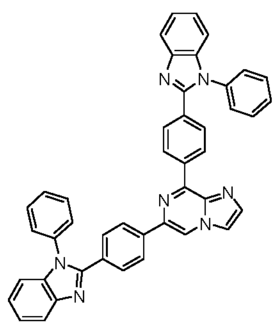
(c),

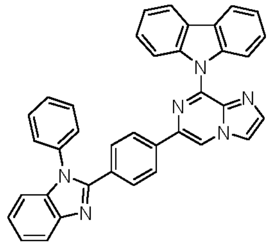 (d), 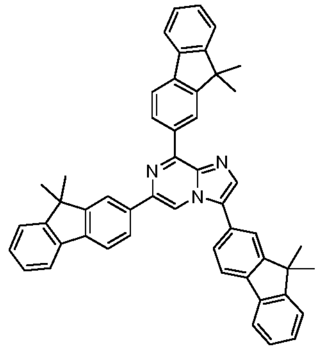 (e),

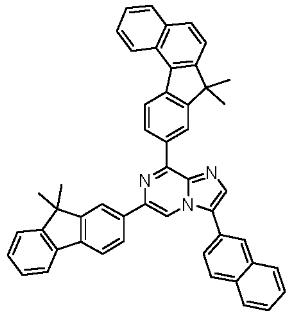 (f),

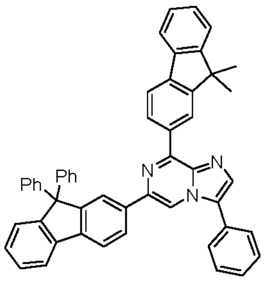 (g), 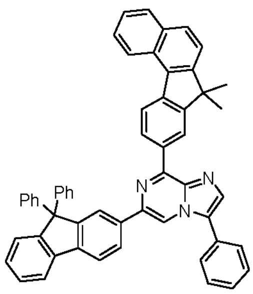 (h),

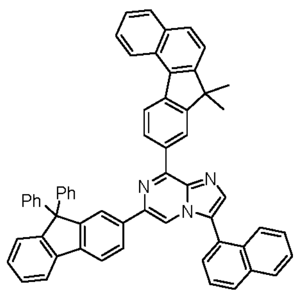 (i), 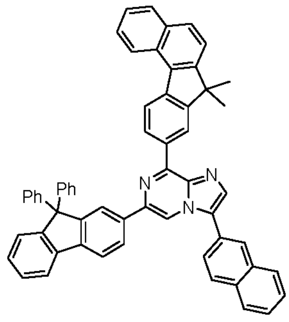 (j), 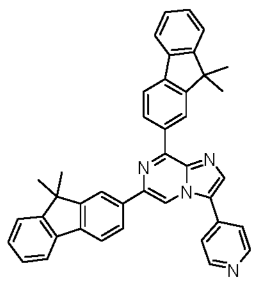 (k),

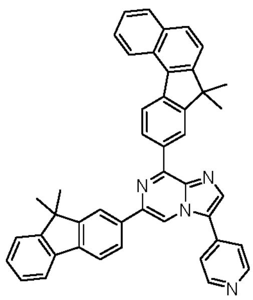 (l), 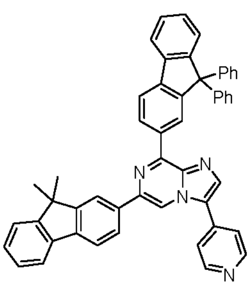 (m), 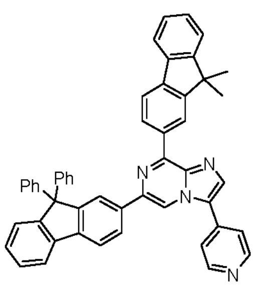 (n),

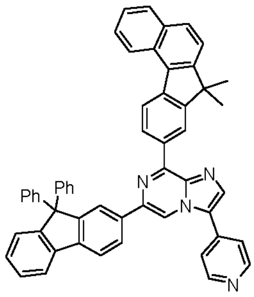 (o), 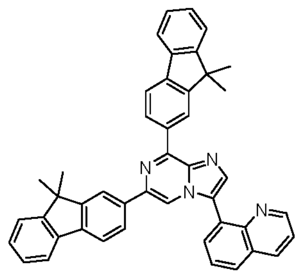 (p), 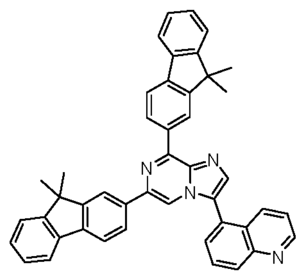 (q),

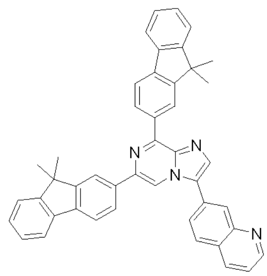 (r), 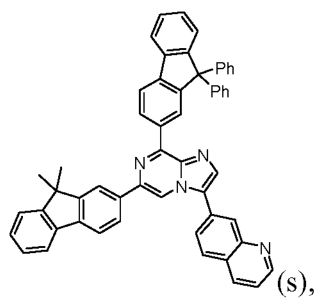 (s), 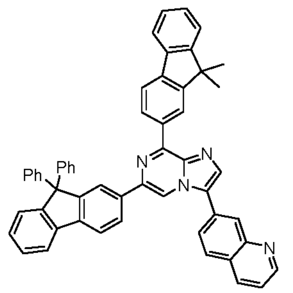 (t),

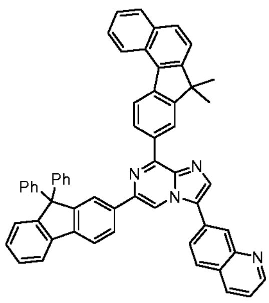 (u), 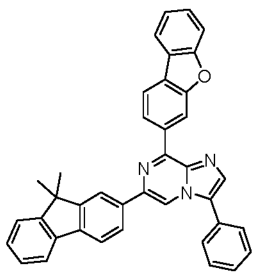 (v), 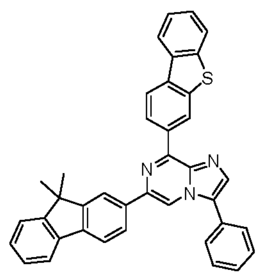 (w),

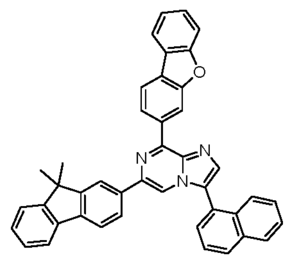 (x), 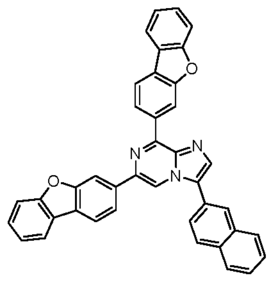 (y), 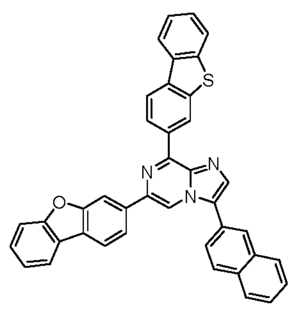 (z),

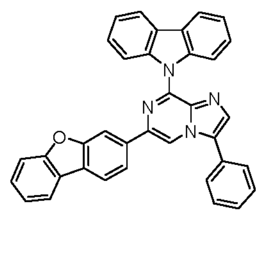 (a1), 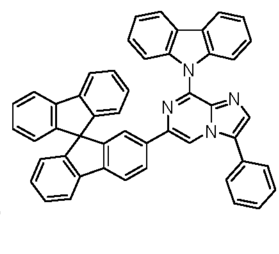 (b1), 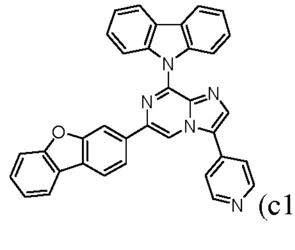 (c1),

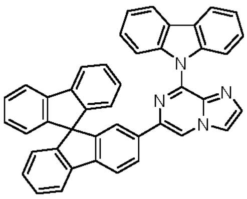 (d1), 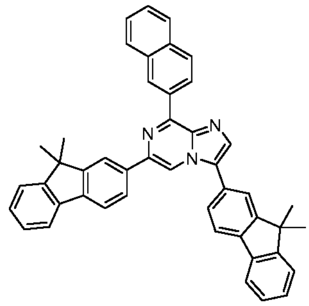 (e1), 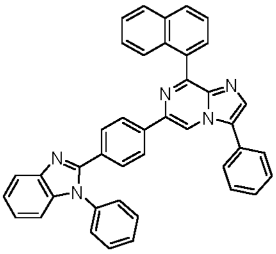 (f1),

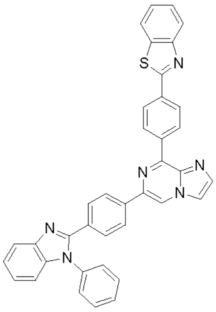 (g1), 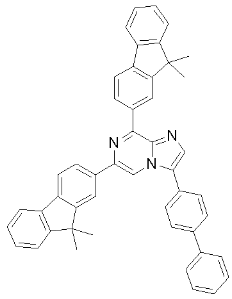 (h1), 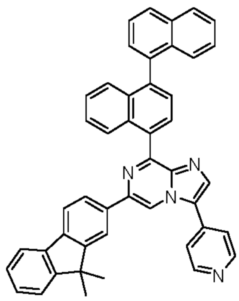 (i1),

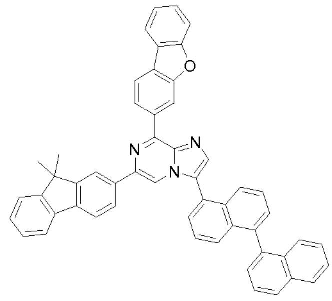 (j1), 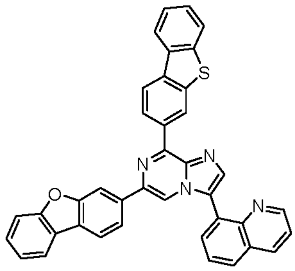 (k1),

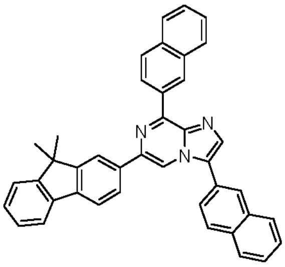 (l1), 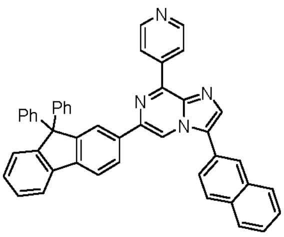 (m1),

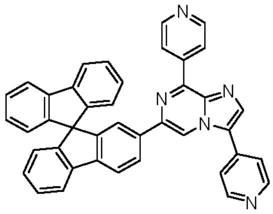 (n1), 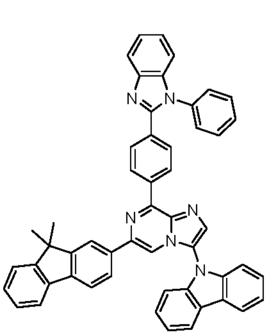 (o1), 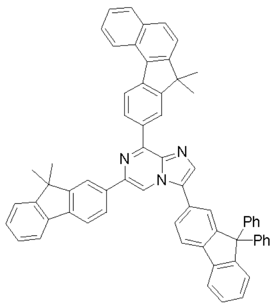 (p1),

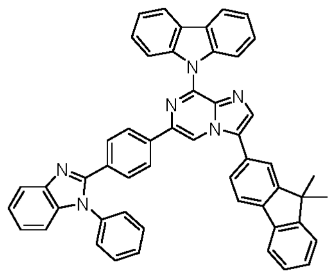 (q1), 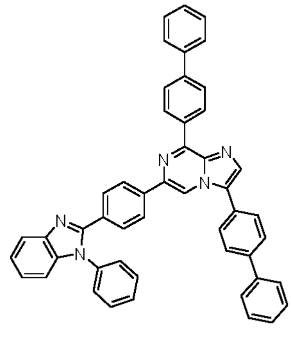 (r1),

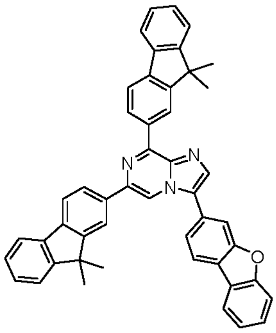 (s1), 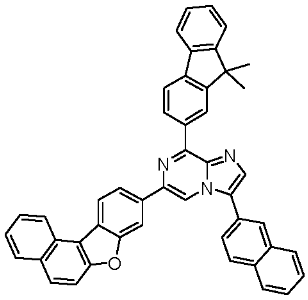 (t1), 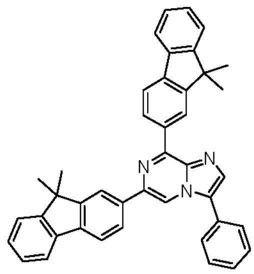 (u1),

or 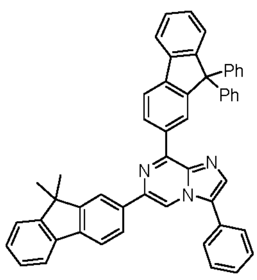 (v1).

2. An article comprising at least one component formed from the composition of claim 1.

3. An electronic device comprising at least one component formed from the composition of claim 1.

## Patentansprüche

1. Zusammensetzung, umfassend mindestens eine Verbindung, die ausgewählt ist aus Folgenden (a) bis (v1):

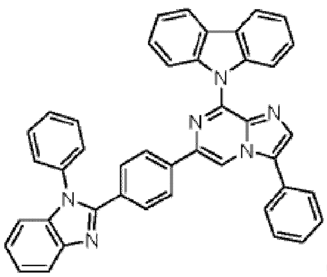 (a), 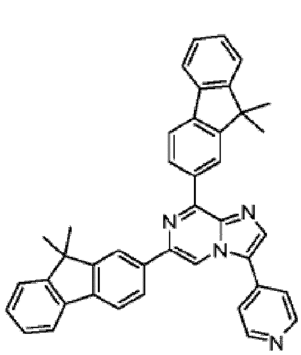 (b), 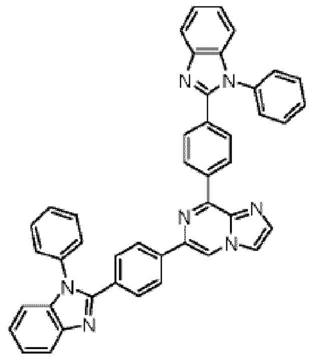 (c),

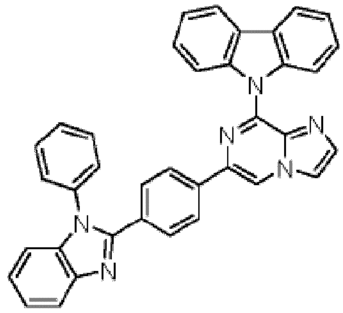 (d),

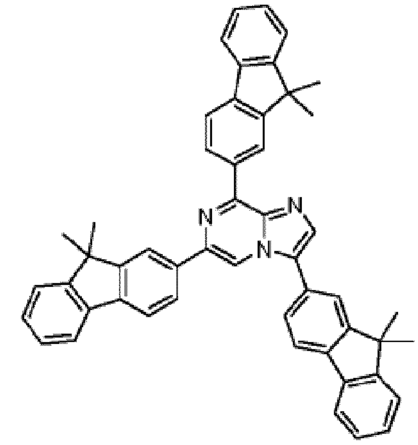 (e),

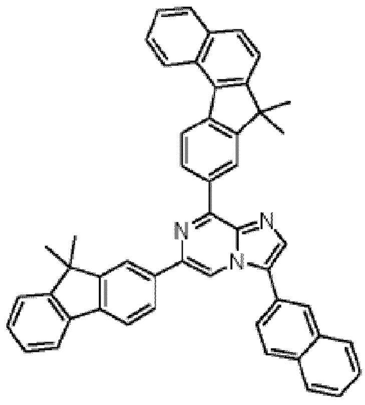 (f),

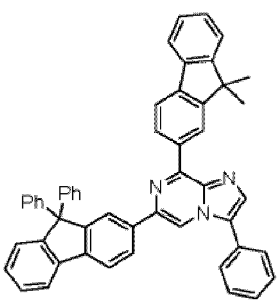 (g),

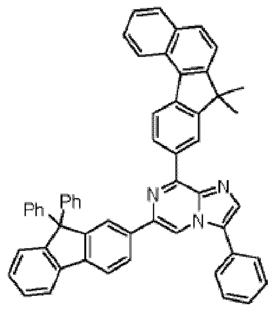 (h),

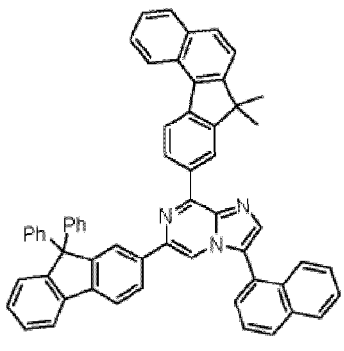 (i),

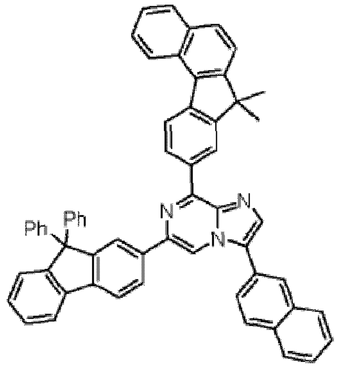 (j),

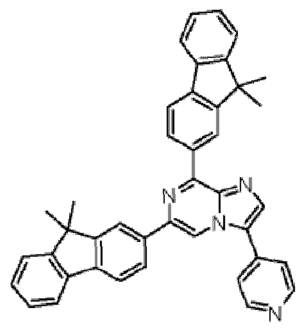 (k),

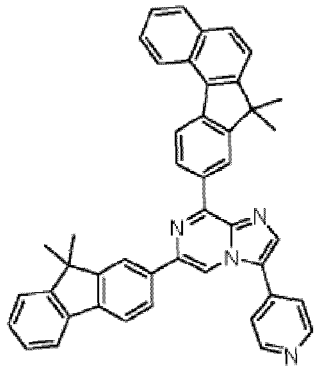 (l), 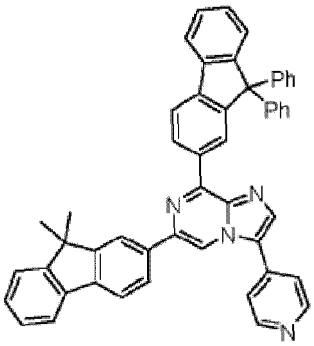 (m), 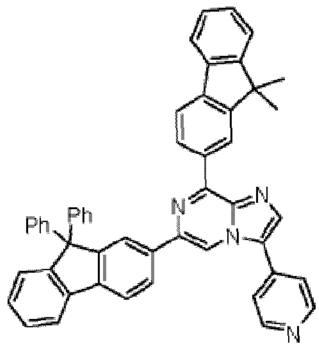 (n),

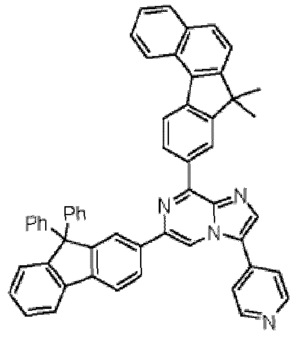 (o), 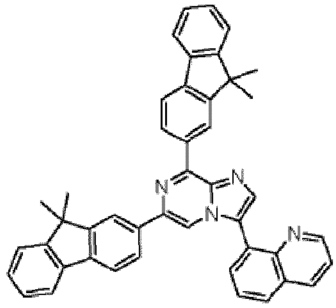 (p), 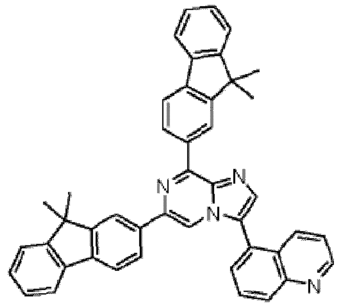 (q),

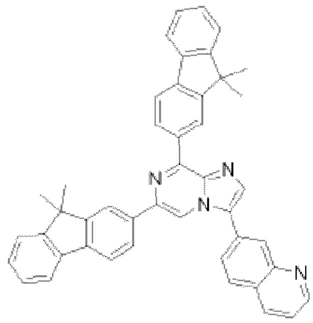 (r), 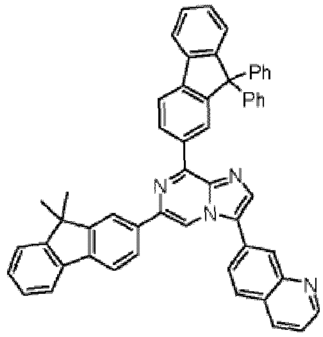 (s), 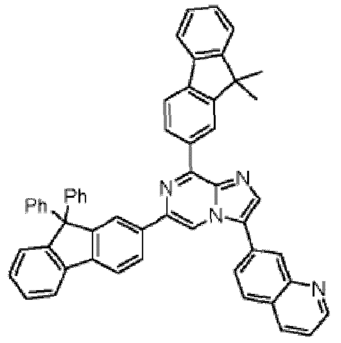 (t),

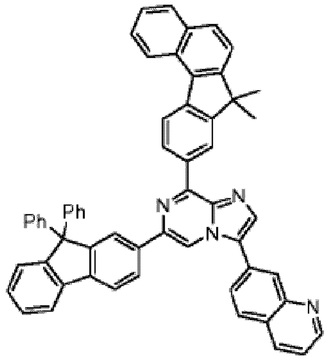 (u), 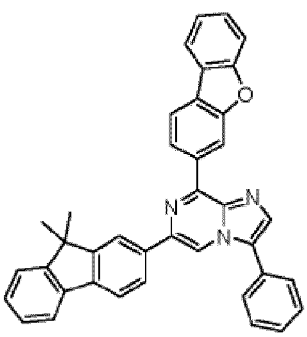 (v), 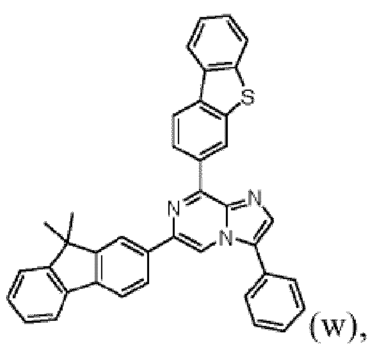 (w),

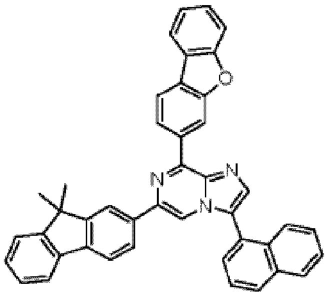 (x), 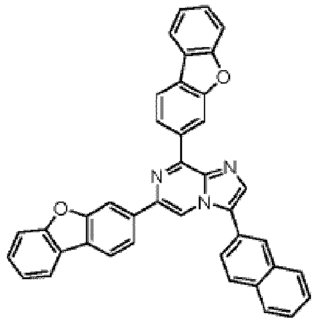 (y), 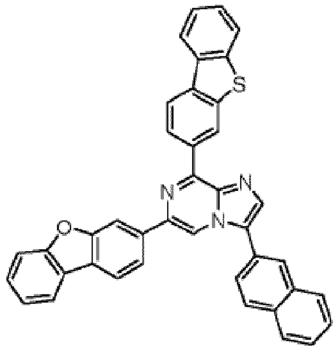 (z),

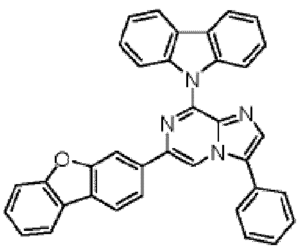 (a1), 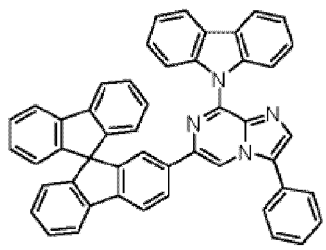 (b1), 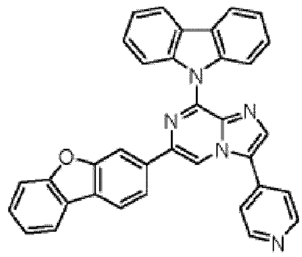 (c1),

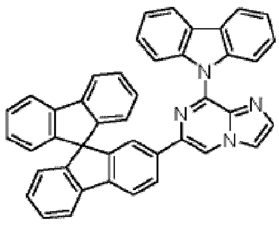 (d1), 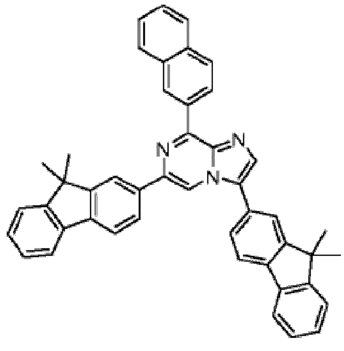 (e1), 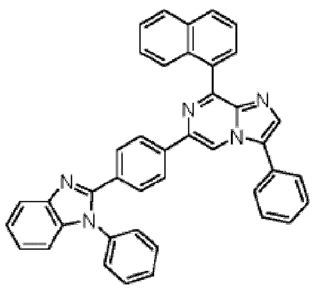 (f1),

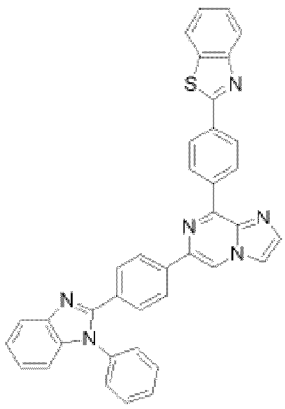 (g1), 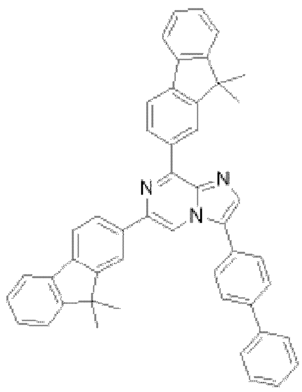 (h1), 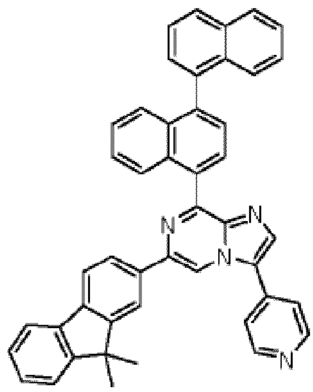 (i1),

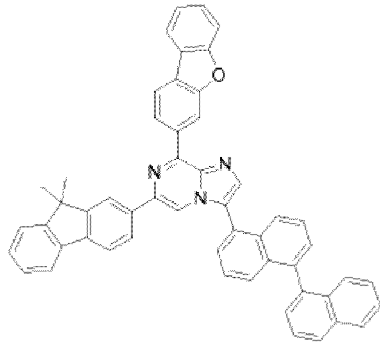 (j1), 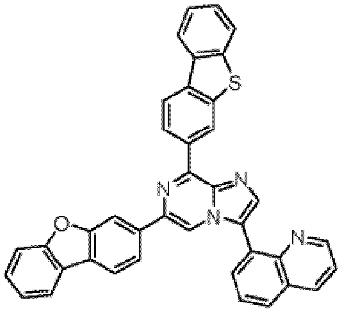 (k1),

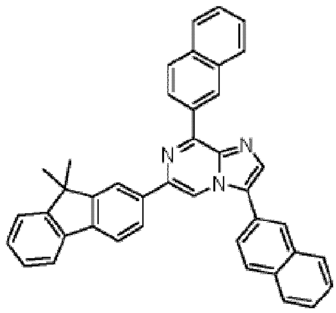 (l1), 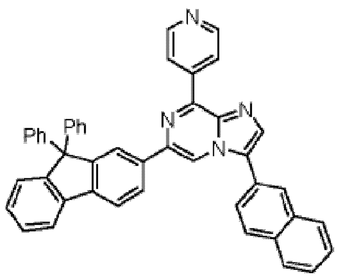 (m1),

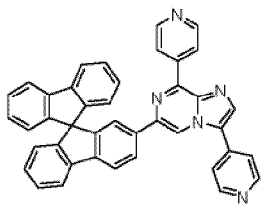 (n1), 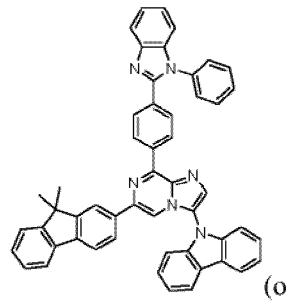 (o1), 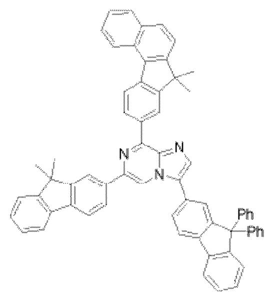 (p1),

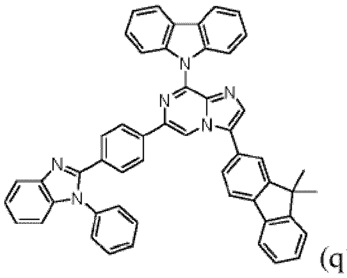 (q1), 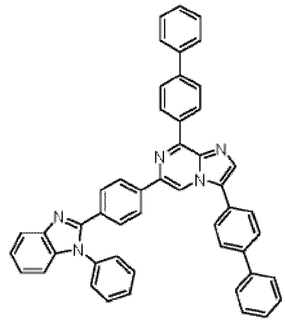 (r1),

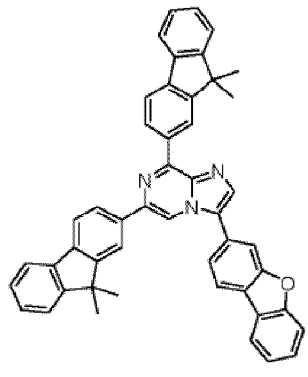 (s1), 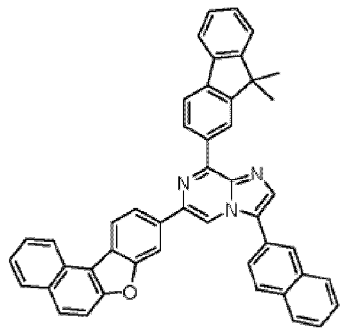 (t1), 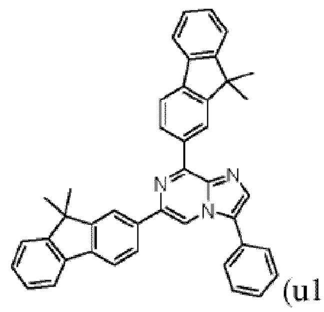 (u1),

oder

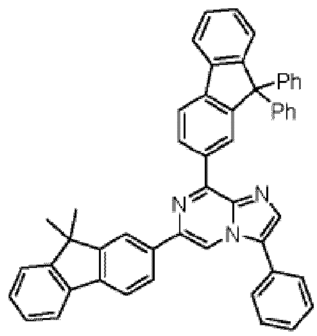 (v1).

2. Artikel, umfassend mindestens eine Verbindung, die aus der Zusammensetzung nach Anspruch 1 gebildet ist.

3. Elektronisches Gerät, umfassend mindestens eine Verbindung, die aus der Zusammensetzung nach Anspruch 1 gebildet ist.

## Revendications

1. Composition, comprenant au moins un composé choisi parmi les composés (a) à (v1) suivants:

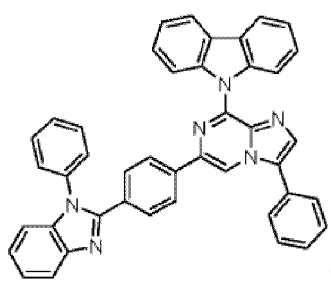 (a), 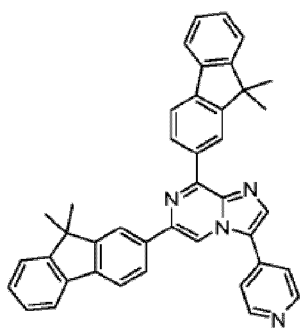 (b), 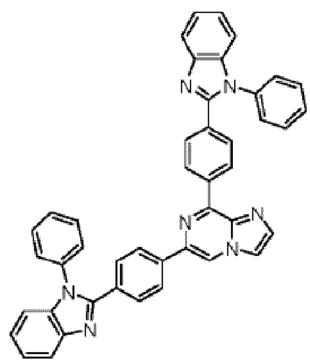 (c),

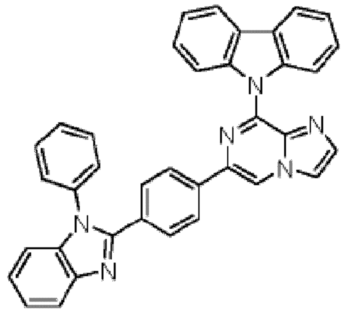 (d),

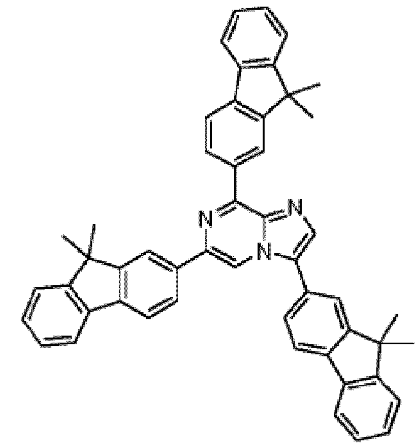 (e),

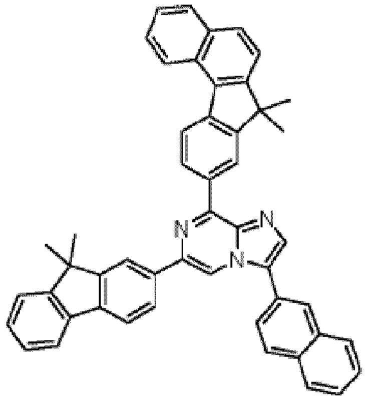 (f),

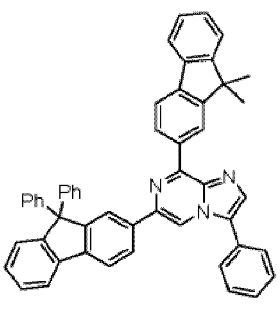 (g),

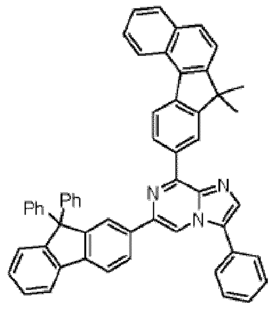 (h),

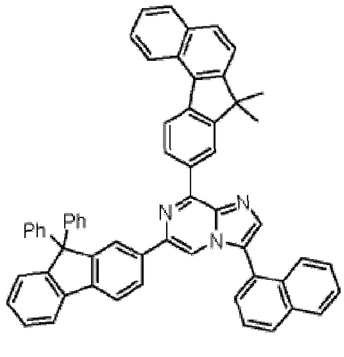 (i),

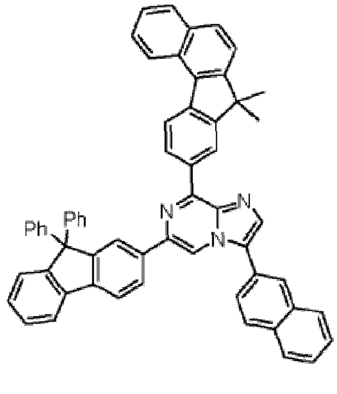 (j),

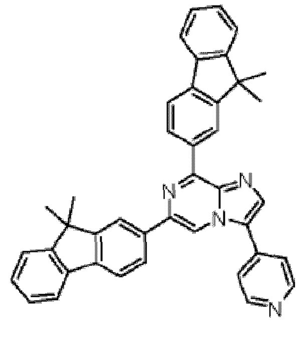 (k),

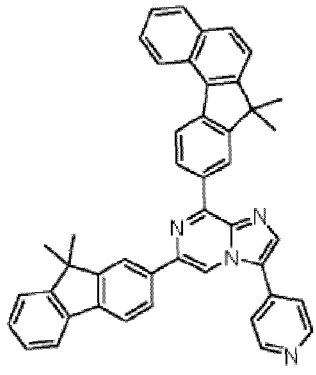 (l),

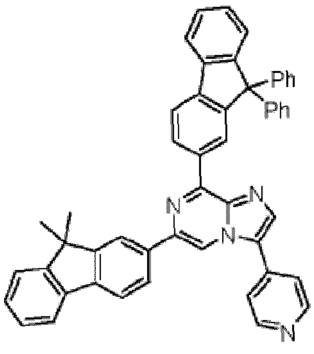 (m),

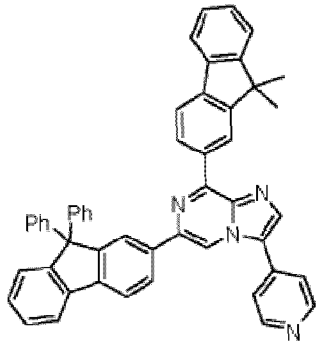 (n),

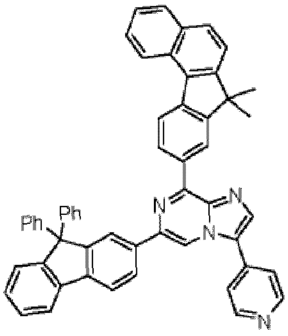 (o),

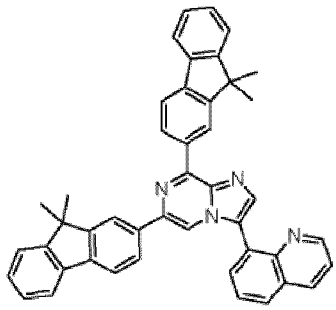 (p),

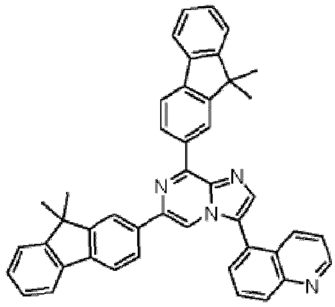 (q),

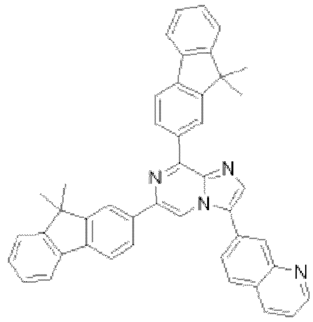 (r),

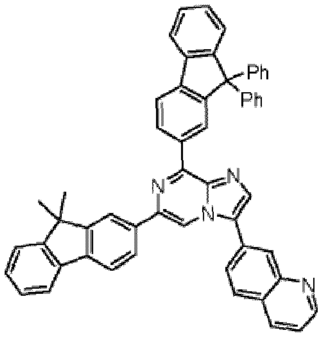 (s),

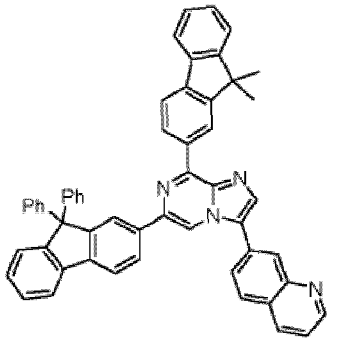 (t),

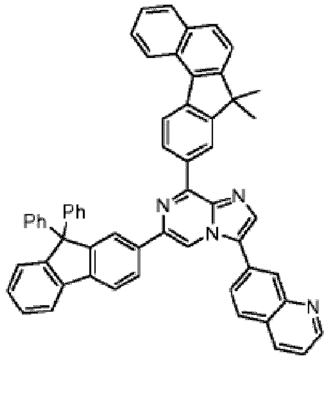 (u),

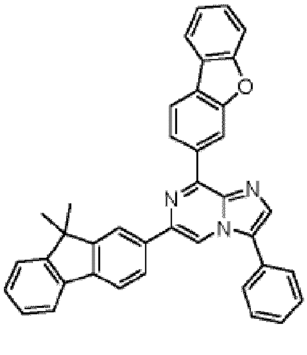 (v),

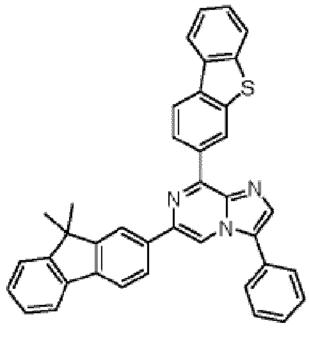 (w),

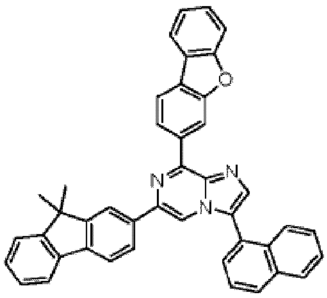 (x), 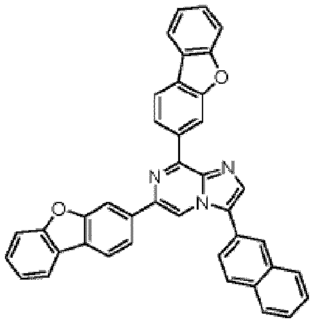 (y), 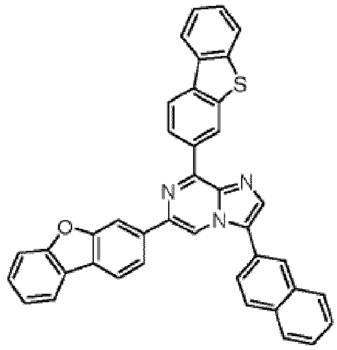 (z),

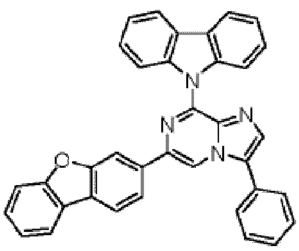 (a1), 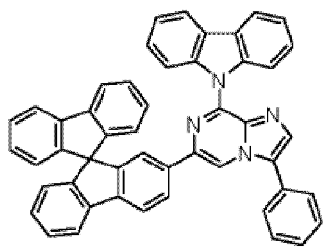 (b1), 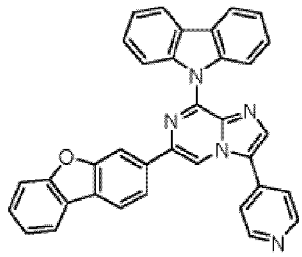 (c1),

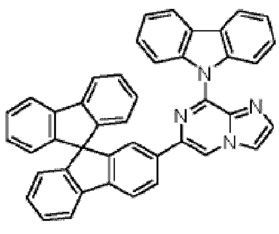 (d1), 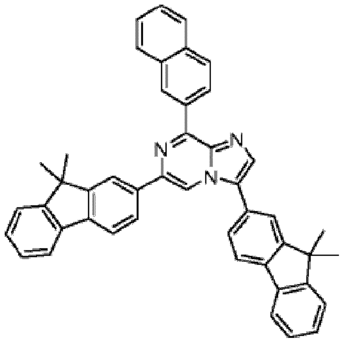 (e1), 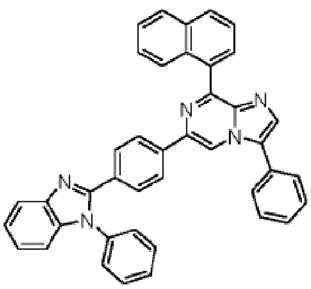 (f1),

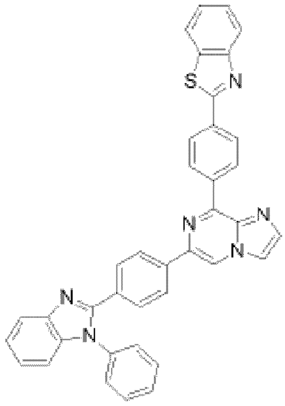 (g1), 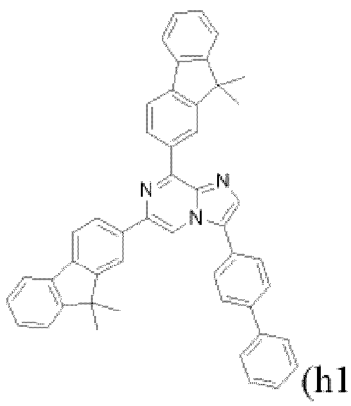 (h1), 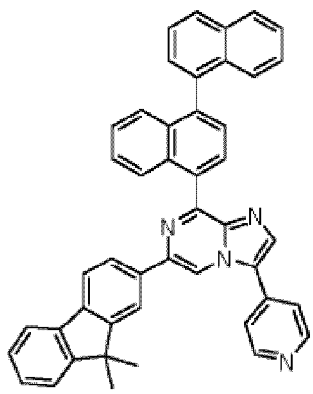 (i1),

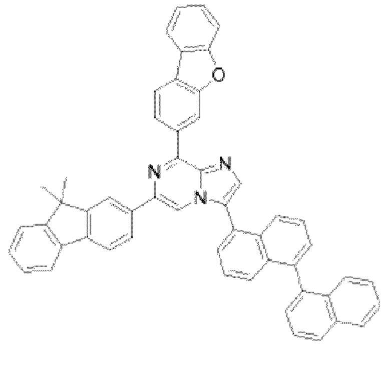 (j1), 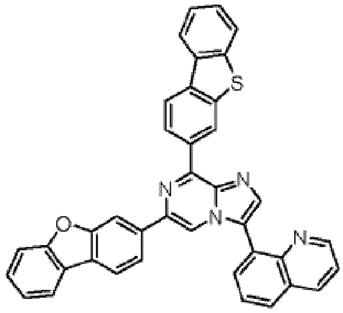 (k1),

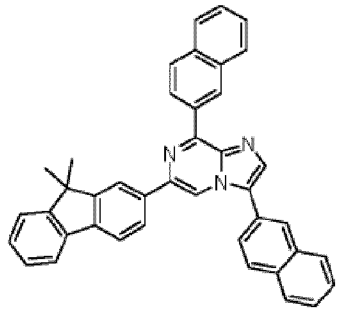 (l1), 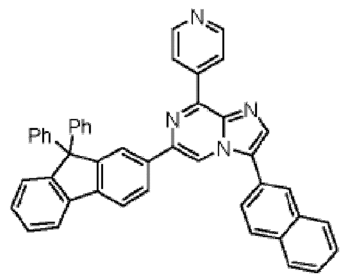 (m1),

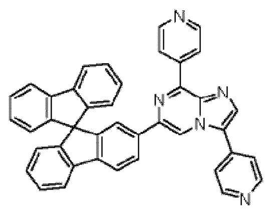 (n1), 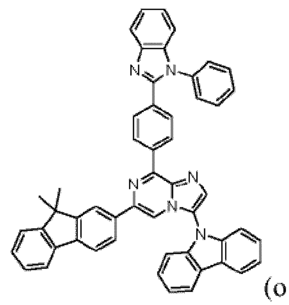 (o1), 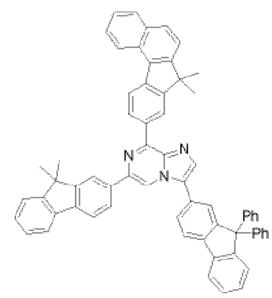 (p1),

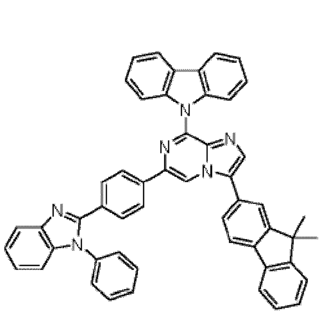 (q1), 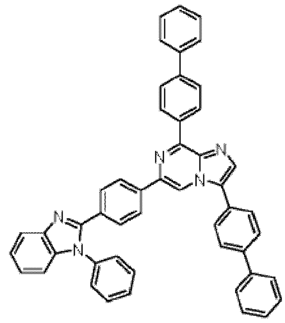 (r1),

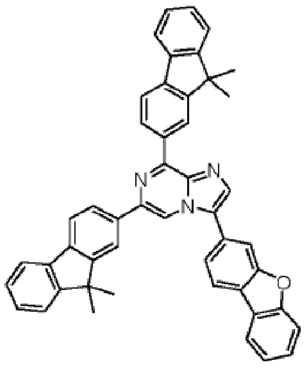
(s1),
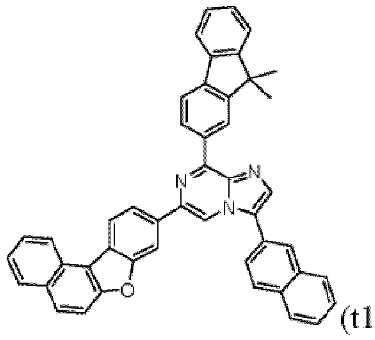
(t1),
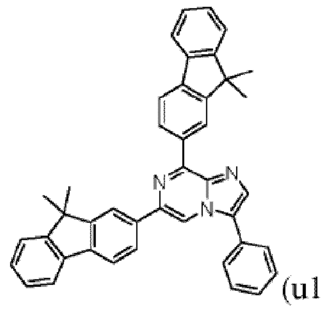
(u1),

ou

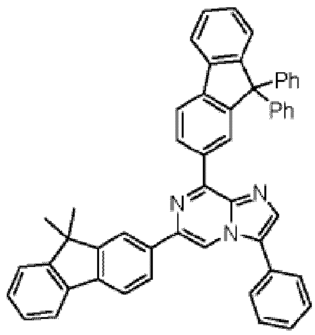
(v1).

2. Article, comprenant au moins un composé formé à partir de la composition de la revendication 1.

3. Appareil électronique comprenant au moins un composé formé à partir de la composition de la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62184948
- US 7867629 B
- US 7745016 B2
- US 20110227058 A1
- US 7745016 B
- EP 406762 A2
- JP 2001043978 A
- CN 104650088
- CN 104650089
- CN 104672240
- CN 104650116
- US 2013299794 A


### Non-patent literature cited in the description


- Graphical representation standards for chemical structural diagrams. *Pure Appl. Chem.,* 2008, vol. 80, 277
- **FRISCH, M.J. et al.** Gaussian 09, Revision A.02. Gaussian, Inc, 2009
- **BECKE, A.D.** *J. Chem. Phys.,* 1993, vol. 98, 5648
- **LEE, C. ; YANG, W. ; PARR, R.G.** *Phys. Rev B,* 1988, vol. 37, 785
- **MIEHLICH, B. ; SAVIN, A. ; STOLL, H. ; PREUSS, H.** *Chem. Phys. Lett.,* 1989, vol. 157, 200
- **DITCHFIELD, R. ; HEHRE, W.J. ; POPLE, J.A.** *J. Chem. Phys.,* 1971, vol. 54, 724
- **HEHRE, W.J. ; DITCHFIELD, R. ; POPLE, J.A.** *J. Chem. Phys.,* 1972, vol. 56, 2257
- **GORDON, M.S.** *Chem. Phys. Lett.,* 1980, vol. 76, 163
- *J. Phys. Chem. A,* 2003, vol. 107, 5241-5251
- *CHEMICAL ABSTRACTS,* 887402-92-8
- *CHEMICAL ABSTRACTS,* 1242056-42-3
- *CHEMICAL ABSTRACTS,* 1207336-74-0
- *CHEMICAL ABSTRACTS,* 1228525-73-2
- *CHEMICAL ABSTRACTS,* 2085-33-8
- *CHEMICAL ABSTRACTS,* 850918-68-2